# EUROPEAN PATENT APPLICATION

(11) **EP 4 112 731 A1**
(43) Date of publication of application: **04.01.2023**
(21) Application number: 21183218.3
(22) Date of filing: 01.07.2021
(51) Int. Cl.: C12N 15/86

(54) **SYSTEM FOR HIGH-LEVEL RAAV PRODUCTION**

(71) Applicant: Charité - Universitätsmedizin Berlin, 10117 Berlin (DE)
(72) Inventor: Weger, Stefan, 14532 Kleinmachnow (DE); Heilbronn, Regine, 12207 Berlin (DE)
(74) Representative: Moré, Solveig Helga

(57) **Abstract**

The present invention is directed to novel production methods for recombinant adeno-associated viral (AAV) vectors or AAV virus-like particles for use in research as well as therapeutic applications. In particular, the invention discloses an advantageous method for producing recombinant adeno-associated virus (rAAV) vectors or AAV virus-like particles wherein a cell is infected with a first recombinant adenoviral vector encoding the AAV Rep proteins, and a second recombinant adenoviral vector encoding the AAV Cap proteins. The cell is further provided with a transgene-vector harboring an expression cassette flanked by AAV inverted terminal repeats (ITRs) comprising a transgene. The invention further discloses a kit or a composition for producing an rAAV vector according to the method of the invention that comprises the transgene vector, the first recombinant adenoviral vector encoding the AAV Rep proteins, and the second recombinant adenoviral vector encoding the AAV Cap proteins. The method, kits and composition of the invention allow for an increased production of AAV vector particles in a stable system.

## Description

The present invention is directed to novel production methods for recombinant adeno-associated viral (rAAV) vectors or AAV virus-like particles, e.g., for use in research as well as therapeutic applications. In particular, the invention discloses an advantageous method for producing recombinant adeno-associated virus (rAAV) vectors or AAV virus-like particles wherein a cell is infected with a first recombinant adenoviral vector encoding the AAV Rep proteins, and a second recombinant adenoviral vector encoding the AAV Cap proteins. The cell is further provided with a transgene-vector harboring an expression cassette flanked by AAV inverted terminal repeats (ITRs) comprising a transgene. The invention further discloses a kit or a composition for producing an rAAV vector according to the method of the invention that comprises the transgene vector, the first recombinant adenoviral vector encoding the AAV Rep proteins, and the second recombinant adenoviral vector encoding the AAV Cap proteins. The method, kits and composition of the invention allow for an increased production of AAV vector particles in a stable system.

Genetically modified viruses are very well suited as vectors for the transfer of genes into human cells in the context of gene therapy. In addition to adenoviral and retroviral vectors, recombinant vectors derived from adeno-associated viruses (rAAV) have gained increasing importance. rAAV vectors have shown excellent efficacy, a very good safety profile, high transduction rates and stable vector persistence combined with long-lasting expression in preclinical animal models and clinical trials for various monogenic diseases. These properties have boosted the use of rAAV in gene transfer protocols for the treatment of a variety of genetic diseases, both in preclinical as well as in clinical studies. In clinical studies, at least partial correction of metabolic defects such as the alpha-1 antitrypsin (Flotte et al., 2011, Hum Mol Gent 20) or the lipoprotein lipase deficiency (Gaudet et al., 2013, Gene Ther 20), haemophilia A (Rangarajan et al., 2017 N Engl J Med 377) and B (Nathwani et al., 2014 N. Engl J Med 371), as well as hereditary forms of blindness such as Leber's congenital amaurosis (LCA) (Bennett et al., 2016, Lancet 388) or Leber's hereditary optic atrophy (Feuer et al., 2016, Opthalmology 123) could be achieved. Under the trade name LUXTURNA, rAAV vector-mediated expression of the RPE65 gene in the retina of LCA patients received clinical approval in the US in December 2017 and in the EU in November 2018. A rAAV vector for the treatment of spinal muscular atrophy (SMA) (Mendell et al, 2017 N Engl J Med. 377) received clinical approval in the US in May 2019 and in the EU in July 2020 under the trade name ZOLGENSMA.

The use of rAAV vectors in the clinical setting, especially in the treatment of human diseases involving body-wide gene transfer, requires production and purification systems capable of generating large amounts of highly-purified and infectious vector particles (Clement and Grieger, 2016, Mol Ther Methods Clin Dev 3). The original approach for rAAV production, the co-transfection of HEK293 cells with a mixture of vector and helper plasmids, is still widely used for clinical grade vector preparations (Clement and Grieger, 2016, Mol Ther Methods Clin Dev 3). In brief, one of the plasmid constructs used in the co-transfection method contains the therapeutic transgene flanked by the AAV inverted terminal repeats (ITR), which are required *in cis* for replication and packaging of the vector genome. A second plasmid provides the AAV Rep/Cap cassette *in trans.* The required adenoviral (AdV) helper functions E2a, E4orf6 and the VA RNA are either provided together with the Rep/Cap functions on the second plasmid, as in the pDG plasmid used in many laboratories (Grimm et al., 1998, Gene Ther 26), or separately on a third plasmid. However, the latter then requires the co-transfection of a total of three plasmid constructs. When using HEK293 cells, the additionally required adenoviral functions E1a and E1b are expressed from adenoviral sequences constitutively integrated into the host genome.

Whereas substantial progress has been made in recent years in adapting such transfection-based production systems to suspension cells (Grieger et al., 2016, Mol Ther 24), the transfection step still constitutes a limiting factor regarding vector yield and ease of handling.

Especially for systemic application in clinical trials, the use of rAAV requires particular large quantities of vector particles, in the order of up to 10¹⁶ genomic particles per patient (Clement et al, 2016, Mol Ther Methods Clin Dev 3). However, in the traditional transfection-based manufacturing process for rAAV vectors described above, yields of a maximum of not more than 5.0 x 10⁴ to 1.0 x 10⁵ genomic vector particles per cell used (GP/cell, alternatively, vg/cell (viral genomes/cell), the so-called "burst size") before and 2.0 to 3.0 x 10⁴ GP/cell after purification can be achieved (Clement et al, 2016). Despite the aforementioned advances in adaptation to suspension cells (Grieger et al., 2016), these methods are furthermore still largely limited to adherent cells. Therefore, achieving a vector preparation of 10¹⁶ genomic particles at maximum yield in the production process requires approximately 3.0 to 5.0 x 10¹¹ cells, thus rendering the traditional transfection-based manufacturing method highly time-consuming and costly.

An alternative to the transfection-based production of rAAV in mammalian cells is the production in insect cells. Insect cells serve as host cells for the subfamily of densoviruses (Denso-virinae) within the parvoviruses. Densoviruses replicate their genome by a very similar mechanism as AAV (Ding et al., 2002, J Virol 76). It was shown that Sf9 cells isolated from *Spodop-tera frugiperda* are able to support Rep protein-mediated replication of an AAV-ITR flanked vector genome without additional helper functions (Urabe et al., 2002, Hum Gene Ther 13). This allowed the establishment of the first infection-based rAAV production procedures in Sf9 suspension cultures using multiple recombinant baculoviruses to deliver the AAV-ITR flanked transgene cassette and the AAV Rep and Cap proteins (Urabe et al., 2002). This original infection-based method has undergone several further developments such as stable insect lines with inducible expression of AAV Rep and Cap proteins (Aslanidi et al., 2009, Proc Natl Acad Sci 106, Mietzsch et al., 2014, Hum Gene Ther 25) and is now successfully used for the production of rAAV vectors for clinical trials (Clement et al, 2016). However, the rAAVs produced in insect cells appear to exhibit poorer transduction characteristics as compared to rAAV produced in human cells (Rumachik et al., 2020 Molecular Therapy Methods & Clinical Development 18), probably due to differential post-translational modifications of the capsid proteins.

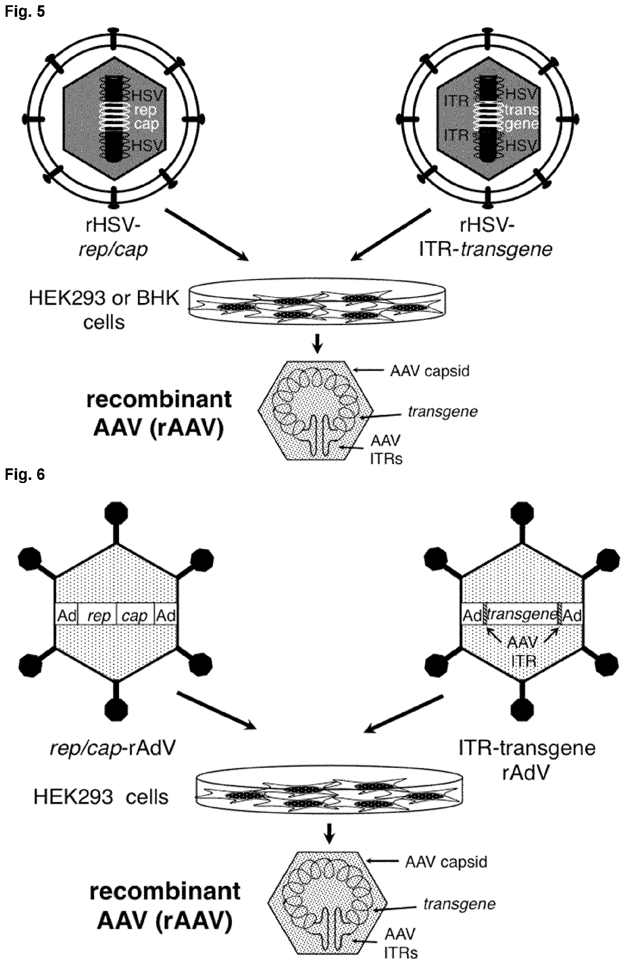

In addition to the rAAV production methods based on infection of insect cells with baculoviruses, there have been efforts for some time to provide the AAV-ITR flanked transgene cassette as well as the AAV Rep and Cap functions in mammalian cells with the help of recombinant helper viruses derived from adenoviruses or herpes simplex viruses (HSV). After infection, these viruses can concurrently provide the helper functions required for rAAV production. Such infection-only rAAV production systems have already been successfully implemented for HSV (Conway et al., 1999, Gene Ther 6) and adapted to the large-scale rAAV production in suspension cells required for clinical trials (Clement et al., 2009, Hum Gene Ther 20, Thomas et al., 2009, Hum Gene Therapy 20) (Figure 5). However, HSV is less well characterized than adenoviruses (Ad) as AAV helper virus. Furthermore HSV vectors are less stable than Ad vectors and more difficult to amplify to high titer levels.

On the other hand, the establishment of rAAV production systems based exclusively on recombinant adenoviruses (rAd) (Figure 6) has not yet been successful. This is despite the fact that adenoviruses are the best-studied helper viruses for AAV.

One of the required components, a rAd vector with the AAV-ITR-flanked transgene cassette, can be well propagated and, in combination with stable cell lines for the expression of the AAV-2 Rep and Cap proteins, can also be successfully used for large-scale rAAV production (Zhang et al., 2009, Hum Gene Ther 20). However, the second required component, a recombinant adenovirus comprising the AAV *rep* and *cap* genes, could not be propagated at all or was genetically unstable and thus is deemed unsuitable for adaptation to the large scale (Zhang et al., 2001, Gene Ther 8).

The use of rAAV vectors in the clinical setting, especially in the treatment of human diseases involving body-wide gene transfer, however requires production and purification systems capable of generating large amounts of highly-purified and infectious vector particles. Therefore, in light of the state of the art, the present inventors addressed the problem of providing a novel and highly efficient method for up-scaling the production of recombinant AAV vectors, e.g., for pre-clinical and clinical requirements.

The problem is solved by the present invention, in particular by the subject matter of the claims.

In a first aspect, the present invention is directed to a method for producing recombinant adeno-associated virus (rAAV) vectors or AAV virus-like particles, wherein the method comprises infecting a cell with
a) a recombinant adenoviral rep-vector comprising an expression cassette comprising the AAV rep gene operably linked to a promoter, wherein the rep gene does not comprise a RIS-Ad, and wherein the rep-vector does not encode the AAV Cap proteins, and
b) a recombinant adenoviral cap-vector comprising an expression cassette comprising the AAV cap gene operably linked to a promoter that is not the wild-type p40 promoter, wherein said cap-vector does not encode the AAV Rep proteins,
and further providing c) a transgene-vector harboring an expression cassette comprising a transgene operably linked to a promoter, wherein the expression cassette is flanked by AAV inverted terminal repeats (ITR),
wherein a) and c) are optional for production of AAV virus-like particles.

In a preferred aspect, the invention provides a method for producing recombinant adeno-associated virus (rAAV) vectors, wherein the method comprises infecting a cell with
a) a recombinant adenoviral rep-vector comprising an expression cassette comprising the AAV *rep* gene operably linked to a promoter, wherein the *rep* gene does not comprise a RIS-Ad, and wherein the *rep*-vector does not encode the AAV Cap proteins, and
b) a recombinant adenoviral cap-vector comprising an expression cassette comprising the AAV *cap* gene operably linked to a promoter that is not the wild-type p40 promoter, wherein said *cap*-vector does not encode the AAV Rep proteins,
and further providing c) a transgene-vector harboring an expression cassette comprising a transgene operably linked to a promoter, wherein the expression cassette is flanked by AAV inverted terminal repeats (ITR).

Alternatively, the invention provides a method for producing recombinant AAV virus-like particles, wherein the method comprises infecting a cell with a recombinant adenoviral *cap-*vector comprising an expression cassette comprising the AAV *cap* gene operably linked to a promoter that is not the wild-type p40 promoter, wherein said cap-vector does not encode the AAV Rep proteins.

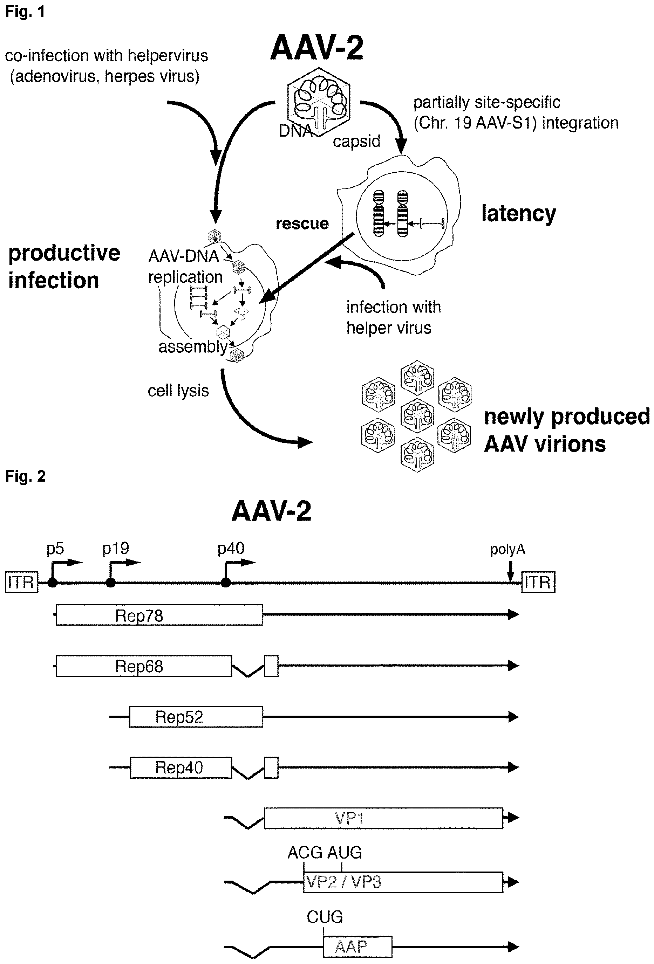

AAVs form the genus Dependoviruses within the family of parvoviruses (Parvoviridae), a family of single-stranded DNA viruses with a non-enveloped, icosahedral capsid. Their productive infection cycle with replication of AAV DNA and assembly of new infectious virus particles depends on the presence of a suitable helper virus, for example adenovirus or herpes simplex virus (HSV), hence its name and taxonomy classification (Figure 1). In the absence of helper viruses, AAV is able to establish a latent infection. *In vitro,* this often results in integration of the viral DNA into the genome of the culture cells, whereas, *in vivo,* the AAV DNA is thought to predominantly exist in an extrachromosomal state as a stable episome. So far, AAV could not be causally linked to a known disease in humans.

In the context of the invention, the term "recombinant AAV" refers to an AAV whose genome has been modified by artificial means, e.g., by recombinant DNA technology via genetic engineering to obtain an AAV that comprises a transgene and may be used as a vector e.g., in gene therapy.

By comparison, the term "AAV virus-like particle" refers to a molecule that resembles an actual AAV, but does not contain any viral genetic material and thus is non-infectious. Virus-like particles are assembled from viral structural proteins, e.g., proteins that form the viral capsid. AAV virus-like particles may serve as delivery systems for genes or other therapeutic agents, or may be used as vaccines. The empty AAV-virus like particles produced by the method of the invention may also serve as decoys for AAV-specific antibodies produced by a patient's immune system, and they may thus beneficially affect AAV-mediated gene transfer during gene therapy.

In the context of the invention, the term "producing" rAAV vectors or AAV virus-like particles refers to their generation by exploiting the protein biosynthesis machinery of a producer or packaging cell. Producing may also mean amplifying rAAV, e.g., amplifying a low amount of a starter rAAV to titers sufficiently high for applications at, e.g., the clinical level, as described herein.

### The AAV genome

The genome of the best characterized AVV serotype AAV-2 has a length of about 4.7 kb and contains as essential elements two open reading frames (ORFs) for the four regulatory (Rep) proteins Rep78, Rep68, Rep52 and Rep40 and the three capsid (Cap) proteins VP1, VP2 and VP3 flanked by two inverted terminal repeats (ITRs) of 145 bp each (Figure 2).

In the context of the invention, the AAV vector produced by the disclosed method is preferably based on the AAV-2 serotype. Accordingly, AAV-2 is used as the reference serotype throughout the invention. Unless explicitly stated otherwise, all references to the AAV genome, individual AAV proteins as well as nucleic acid or amino acid sequences of AAV are made with regard to AAV-2.The person skilled in the art can easily transfer all the information given with regard to AAV-2 genome to other AAV serotypes.

The rep and cap genes of AAV-2 partially overlap, as they share a common intron. The large Rep proteins Rep78 and Rep68 are expressed under the control of the endogenous p5 promoter (Redemann et al., 1989, J Virol 63) and play an essential role in AAV-2 DNA replication via their sequence-specific DNA binding to the AAV-ITRs (McCarty et al., 1994 J. Virol 68). In addition, they stimulate the expression of the small Rep proteins Rep52/Rep40 and the capsid proteins in the presence of the helper virus by binding to other elements in the AAV-2 genome and a number of cellular transcription factors (Pereira and Muzyczka, 1997, J Virol 71). The small Rep proteins Rep52 and Rep40 are expressed by the p19 promoter and are required for the insertion of newly formed single-stranded DNA into pre-formed capsids during DNA replication (King et al., 2001, Embo J 20)). Rep78 and Rep52 are encoded by unspliced transcripts, whereas the transcripts for the shorter variants Rep68 and Rep40 excise the intron localized in the 3' part of the rep gene. Replication studies show that one of the large Rep proteins Rep78 or Rep68 together with one of the small Rep proteins Rep52 or Rep40 is sufficient for replication of AAV-2 DNA and virus production (Ward et al., 1994, J Virol 68).

The three capsid (Cap) proteins, VP1, VP2 and VP3, are produced under the control of the p40 promoter via the use of alternatively spliced transcripts and translation initiation codons (Becerra et al., 1988, J Virol 68, Muralidhar et al., 1994, J Virol 68) in the stoichiometry of 1:1:10 (Xie et al., 2002, Proc Natl Acad Sci 99), which is also present in the final capsid. Of the predominantly formed splice variant, in which the same intron in the 3' part of the *rep* gene is excised as in the expression of Rep68 and Rep40, the synthesis of VP2 is triggered by a non-canonical ACG start codon and that of the main capsid protein VP3 by a regular AUG start codon further downstream. The less abundant alternatively spliced p40 transcript uses the same splice donor site but a more upstream splice acceptor site, so that this transcript still contains the AUG start codon for VP1. Capsid structures are assembled above a critical concentration of capsid proteins with the involvement of chaperones such as the AAV-encoded assembly activating protein (AAP). AAP is necessary for the formation of infectious virus particles in the AAV-2 serotype and enhances this process in other serotypes (Earley et al., 2017, J Virol 91, Sonntag et al., 2011, J Virol 85, Sonntag et al., 2010, Proc Natl Acad Sci 107). It is encoded by an alternative reading frame in the cap gene. AAV DNA replication and the assembly of finished DNA-containing viral particles are thought to constitute coupled processes.

A major factor in the use of viral vectors such as rAVV is safety, as they must not be harmful to the target organism. Therefore, replication-deficient AVV have to be developed, which can still introduce the therapeutic gene into the target cell, but can no longer replicate in it. This is achieved by removing viral genes that are essential for replication.

Although AAVs are considered apathogenic and depend on helper viruses for productive replication, the coding viral sequences (Rep/Cap) in the recombinant genome are usually completely removed. This is intended on the one hand to make room for the therapeutic transgene, as the maximum packaging capacity for the vector genome is approximately in the order of magnitude of the wild-type genome with a length of 4.7 kb. On the other hand, possible toxic effects of high Rep concentrations should be avoided, as, e.g., for Rep78 in cell culture, a number of inhibitory effects on, inter alia, cell proliferation and the cell cycle have been described. Due to the deletion of the *rep* gene, rAAV vectors also lose the ability to integrate into the genome of the transduced target cell. However, the episomal vector genomes show a very high stability in differentiated cells due to the formation of so-called concatamers.

The inventors found that AAV vector production can be successfully achieved by providing the AAV rep gene and the AAV *cap* gene from two separate recombinant adenoviral vectors, herein referred to as rep-vector and cap-vector or rep-rAdV and cap-rAdV, respectively. They could demonstrate that co-infection of a cell with separate *rep-* and *cap-*rAdVs can functionally replace the Rep/Cap cassette and the adenoviral helper functions of the traditional plasmid-based production method completely. This approach also eliminates the need to produce large quantities of highly purified plasmid DNA, which was necessary in the previous standard transfection process.

### Adenoviral viruses as vectors

Adenovirus refers a family of non-enveloped icosahedral viruses with a linear doublestranded DNA genome of a length of 26-45 kilo base pairs (kbp). Due to their large genome size, uncomplicated generation and the possibility of their amplifying to high titres without the need for complex complementing cell lines, recombinant adenoviruses (rAd) are considered attractive vectors for research applications and gene transfer.

Over the years, rAd vector (rAdV) technology has been continuously advanced to improve its safety and therapeutic potential. Until now, three generations of rAdVs have been designed. Recombinant adenoviral vectors of the first generation still contain all adenoviral genes except for the E1a/E1b and the E3 region that are essential for virus replication. While the E3 region, due to its exclusively immunomodulatory function, is expendable for rAdV propagation in cell culture per se and is also not involved in the helper effect for productive AAV infection, the missing E1a/E1b functions required for rAdV amplification can be provided by, e.g., genetically modified packaging cells such as HEK293 cells. First generation rAdVs have a cloning capacity of about 8.2 kb. Second generation rAdVs further lack the non-structural E2 and E4 genes, which results in an increased cloning capacity of about 12 kb. Finally, third generation rAdVs have all their viral protein coding sequences removed and only retain the adenoviral ITRs and a packaging signal in *cis*. Accordingly, third generation rAdVs rely on co-infection with a helper adenovirus for provision of the viral proteins in *trans.*

First generation rAdVs can be amplified inexpensively to very high titers by simple infection of well-established and easy-to-handle complementing cell lines such as HEK293. Furthermore, the cloning capacity of first generation AdVs is sufficiently large to harbor the AAV *rep* or the *cap* open reading frames. Therefore, in a preferred embodiment, the recombinant adenoviral *rep-* and *cap-vectors* are first generation recombinant adenoviral vectors (rAdVs). However, the *rep-* and *cap*-rAdVs may, in another embodiment, also be second generation rAdVs. The *rep-* and *cap*-rAdVs may also be third generation rAdVs. However, use of third generation rAdVs for the method of the invention would require co-infection with a suitable helper adenovirus as described above, and therefore is less preferable.

The two different rAdVs used for the expression of the AAV Rep and Cap proteins can be produced by any method known from the state of the art. For example, the rAdV may be produced using the AdEasy^{™} Adenoviral Vector System by the company Agilent Technologies, as described in the examples below. A person skilled in the art may however also refer to different rAdV production systems known in the art or may rely on commercially provided custom-made AdV services for obtaining the herein described rep- and cap-rAdVs.

Previous attempts to stably propagate a rAdV expressing both the AAV-2 *rep* and cap genes for the large-scale production of rAAV have failed. The Rep and Cap-expressing rAdVs could either not be replicated or were inherently genetically unstable. Inhibition of adenoviral replication was also observed with rAdVs exclusively containing the AAV rep gene (Carlson et al., 2002, Mol Ther 6, Recchia et al., 1999, Proc Natl Acad Sci USA 96, Recchia et al., 2004, Mol Ther 10, Wang and Lieber, 2006, J Virol 80) and the failure to stably propagate rAd-rep/cap vectors was therefore attributed to Rep protein expression. However, recent studies by Sitaraman *et al.* (Sitaraman et al., 2011, Proc Natl Acad Sci USA 108) and the present inventors have shown that the limited replication of these hybrid AAV/Ad vectors is in large parts due to a *cis* inhibitory sequence in the 3'- part of the AAV-2 rep gene, which was therefore termed RIS-Ad (*Rep inhibition sequence for adenoviral replication*). The RIS-Ad inhibits adenoviral genome replication by a transcription-associated mechanism, apparently mediated by promoter-proximal paused RNA II polymerase complexes (Weger et al, 2016, J Virol 90).

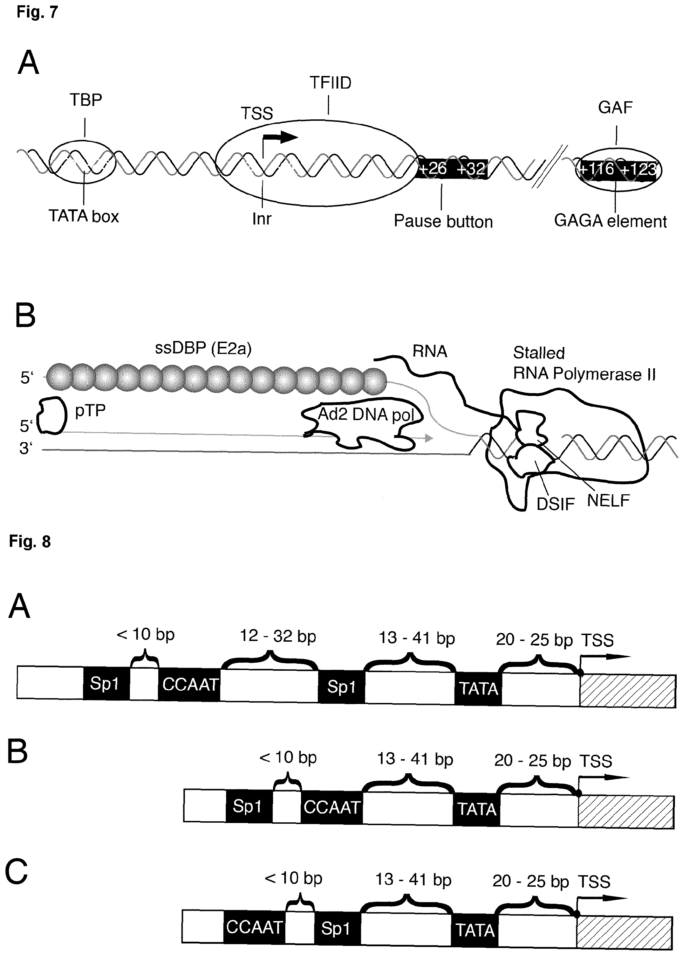

It was shown that the RIS-Ad corresponds to the nucleotide sequence of SEQ ID NO: 2 and spans the nucleotide (nt) positions 1701 to 2186 of the AAV-2 genome (SEQ ID NO: 1). The RIS-Ad is not able to express functional Rep proteins, but still comprises the p40 promoter responsible for the expression of the Cap proteins and large parts of the AAV intron as well as the splice donor site at nt position 1906 of SEQ ID NO. 1, which is essential for the splicing of the transcripts encoding the Cap proteins. Without being bound by theory, it is thought that the inhibition of adenoviral replication in *cis* is based on the expression of short transcripts from the p40 promoter required for expression of the cap transcripts (Weger et al., 2016, J Virol 90). The RNA polymerase II producing these short transcripts pauses directly downstream of the p40 promoter, possibly leading to steric hindrance of replication of the rep-Ad genome in this region (Figure 7).

It was shown that recoding of the entire RIS-Ad can reverse the inhibition of the rep-Ad genome replication. Recoding a nucleic acid refers to a substitution of the triplet codons encoding individual amino acids with synonymous triplet codons so that the translated amino acid sequence remains unchanged. Accordingly, only the primary nucleic acid sequence is altered by the recoding. This can be done since the 64-codon genetic code has redundancy but no ambiguity, i.e., multiple different nucleic acid codons encode for one and the same amino acid. By recoding the RIS-Ad in the 3'-region of the *rep*-gene, the encoded polypeptides, i.e., the AAV Rep proteins, remain unaffected since the amino acid sequence constituting said Rep-proteins is not altered. However, recoding modifies or, preferably, completely disrupts the p40 promoter inside the Rep ORF and thus interferes with RNA polymerase binding to the 3' end of the *rep* gene. Accordingly, inhibition of rAdV replication by the inserted Rep78 cassette can be abolished.

In one embodiment of the invention, the *rep* gene does no longer comprise a RIS-Ad, i.e., the entire RIS-Ad is recoded, e.g., into a nucleic acid sequence that shares 100% sequence identity to SEQ ID NO: 3 as previously described in Sitaraman et al. Alternatively, the recoded region may also have a nucleic acid sequence of at least 5%, at least 10%, at least 20%, at least 30%, at least 40%, at least 50%, at least 60%, at least 70%, at least 80%, at least 90%, at least 95%, at least 96%, at least 97%, at least 98% or at least 99% sequence identity to SEQ ID NO: 3. Preferably, the recoded RIS-Ad has less than 95%, less than 90%, less than 80% or less than 70% sequence identity to SEQ ID NO: 2, i.e. the nucleic acid sequence of the wild-type RIS-Ad.

The inventors also showed that the RIS-Ad can be further narrowed down to a nucleotide sequence spanning nt 1781-2060 of SEQ ID NO: 1 and corresponding to SEQ ID NO: 12. This so-called minimal RIS-Ad may be recoded as described for the RIS-Ad above. The recoded minimal RIS-Ad may therefore have a nucleic acid sequence according to SEQ ID NO: 13. Alternatively, the recoded minimal RIS-Ad may also have a nucleic acid sequence of at least 10%, at least 20%, at least 30%, at least 40%, at least 50%, at least 60%, at least 70%, at least 80%, at least 90%, at least 95%, at least 96%, at least 97%, at least 98% or at least 99% sequence identity to SEQ ID NO: 3. Preferably, the recoded minimal RIS-Ad has less than 95%, less than 90%, less than 80%, less than 70%, less than 60% or less than 50% sequence identity to SEQ ID NO: 12, i.e. the nucleic acid sequence of the wild-type minimal RIS-Ad.

In addition, the inventors found that recoding of only a sub-region of the RIS-Ad spanning nt 1782 to 1916 of SEQ ID NO: 1 is sufficient to abolish the inhibition of *rep*-AdV genome replication. Therefore, in a preferred embodiment, only the region encompassing nt 1782-1916 of SEQ ID NO: 1 is recoded, e.g., into a nucleic acid sequence that shares 100% nucleic acid sequence identity to SEQ ID NO: 4 and is herein referred to as sRep. sRep corresponds to amino acids 488 to 531 of the Rep78 protein (SEQ ID NO: 5). Alternatively, the recoded sub-region of the rep gene may also have a nucleic acid sequence of at least 20%, at least 30%, at least 40%, at least 50%, at least 60%, at least 70%, at least 80%, at least 90%, at least 95%, at least 96%, at least 97%, at least 98% or at least 99% sequence identity to sRep (SEQ ID NO: 4). Preferably, the recoded sub-region of the RIS-Ad spanning nt 1782 to 1916 of SEQ ID NO: 1 has less than 80%, less than 70%, less than 60% or less than 50% sequence identity to the region of nt 1782 to 1916 of SEQ ID NO: 1.

Of note, recoding of the RIS-Ad or the above-described sub-region thereof destroys the splice donor site in the 3' carboxy terminus of the rep gene. In consequence, alternative splicing of rep transcripts is abolished. Thus, in one embodiment, according to the invention, the two alternative Rep proteins Rep68 and Rep40 are no longer expressed. However, expression of only Rep78 and Rep52 was found to be sufficient for replication of AAV-2 DNA and virus production, thus rendering Rep68 and Rep40 expendable.

Recoding a nucleic acid region does not imply that every single triplet codon in said nucleic acid sequence has to be recoded. In some embodiments, recoding a nucleic acid sequence refers to the recoding of merely one or more selected triplet codons within said sequence. For instance, it may suffice to only recode selected triplet codons within the RIS-Ad, e.g., regions within the p40 promoter that are bound by RNA polymerase or particular transcription factors. In one embodiment, recoding of only nucleotides 1851-1856 of SEQ ID NO: 1 in the 3' region of the AAV *rep* gene harboring the p40 transcription start site (TSS) at nucleotide 1853 may be sufficient to reduce p40 promoter function and at least partially alleviate the inhibition of *rep*-AdV replication. Alternatively, a region of 17 nucleotides encompassing a GGT motif corresponding to nt 1781-1797 of SEQ ID NO: 1 may be recoded. In a preferred embodiment, both the p40 TSS corresponding to nt 1851-1856 of SEQ ID NO: 1 and the GGT motif-encompassing p40 region corresponding to nt 1781-1797 of SEQ ID NO: 1 is recoded while the rest of the RIS-Ad may remain unaltered.

In some embodiments, it may also suffice to recode the Sp1-binding site covering nt positions 1802-1810 of SEQ ID NO: 1 and/or the AP1-binding site spanning nt 1813-1820 of SEQ ID NO: 1 to reverse the inhibition of *rep*-AdV replication. The Sp1-binding site and/or the AP1-binding site may also be recoded in addition to the p40 TSS or the GGT motif-encompassing region spanning nt 1781-1797 of SEQ ID NO: 1. It may also be particularly advantageous to concurrently recode the p40 TSS, the GGT motif encompassing region of nt 1781-1797 of SEQ ID NO: 1, the Sp1-binding site and the AP1-binding site as described herein, while the rest of the RIS-Ad remains unaltered. This way, it may also be possible to preserve the splice donor site in the carboxy terminal part of the Rep ORF required for the expression of the alternative Rep proteins Rep68 and Rep40.

In summary, the terms "recoding of the RIS-Ad" or "does not comprise a RIS-Ad" may herein be understood to mean, e.g., the recoding of the entire RIS-Ad corresponding to nt 1701 to 2186 of SEQ ID NO:1, the minimal RIS-Ad corresponding to nt 1781-2060 of SEQ ID NO: 1, the sub-region of the RIS-Ad corresponding to nt 1782-1916 of SEQ ID NO: 1, or merely selected codons of the RIS-Ad encompassing, e.g., the p40 TSS and/or individual transcription factor binding sites as described herein.

Recoding of the RIS-Ad should reduce the activity of the physiological p40 promoter in the 3' region of the Rep ORF to an extent that the p40 promoter no longer interferes with the replication of the *rep*-AdV. In the context of the invention, this is achieved when recoding of the RIS-Ad reduces the activity of the p40 promoter to at most 30% of that of the wild type p40 promoter. Preferably, the activity of the p40 promoter after recoding of the RIS-Ad is even lower than 30% of the activity of the wild type p40 promoter, e.g., the activity of the p40 promoter is preferably reduced to at most 25%, at most 20%, at most 15%, at most 10%, at most 9%, at most 8%, at most 7%, at most 6%, at most 5%, at most 4%, at most 3%, at most 2% or at most 1% of that of the wild type p40 promoter. In a particularly preferred embodiment, recoding of the RIS-Ad results in a complete disruption of p40 promoter function, i.e. the p40 promoter exhibits no measurable transcriptional activity at all.

According to the method of the invention, the 5' end of the AAV-2 *rep* gene provided by the *rep*-AdV is operably linked to a promoter capable of driving expression of the large Rep proteins. A promoter is a DNA element that regulates the timing, rate and location of the transcription of an associated gene. Promoter regions are found upstream of their respective associated genes and are asymmetrically organized to indicate the direction of the gene's transcription into RNA. RNA polymerases initiate the transcription of a gene by binding to the promoter region of said gene. Promoters furthermore comprise a variety of specific sub-regions that may serve as binding sites for transcription factors. Dependent on the type of transcription factor bound to the promoter, transcription of the respective genes is either activated or repressed.

A nucleic acid sequence, e.g., a gene, is considered to be "operably linked" to a promoter when said promoter is positioned in a correct functional location and/or orientation in relation to said nucleic acid sequence to control transcription of that sequence. Naturally, the large AAV-2 Rep proteins Rep78 and Rep68 are transcribed under the control of the AAV-2 p5 promoter positioned at the 5' end of the Rep ORF, whereas the smaller Rep proteins Rep52 and Rep40 are transcribed from the internal p19 promoter present within the Rep ORF. Therefore, in one embodiment, the promoter operably linked to the 5' end of the AAV-2 *rep* gene in the *rep*-AdV is the endogenous AAV-2 p5 promoter. However, the inventors made the surprising discovery that the expression of Rep78 under the control of the physiological AAV-2 p5 promoter resulted in a significant inhibition of *rep*-AdV replication associated with high vector instability. Possibly, the genetic instability of the *rep*-AdV may be due to a relatively strong binding element for the large Rep proteins (RBE) present in the p5 promoter. Therefore, in a preferred embodiment, the physiological promoter operably linked to the AAV-2 *rep* gene inside the rep-AdV is not the AAV-2 p5 promoter but rather a heterologous promoter.

A heterologous promoter refers to a promoter that has been artificially linked to a gene of interest, although said promoter normally does not control the expression of the gene. By using a heterologous promoter, the expression of a transgene may be enhanced or reduced. Alternatively, a heterologous promoter may be an inducible promoter that is only able to ignite gene expression in the presence of a chemical trigger factor. Heterologous promoters may also be cell- or tissue-specific promoters that ensure cell- or tissue-specific expression of their associated genes.

The heterologous promoter of the *rep*-AdV may be any promoter suitable for transgene expression in a viral vector.

However, since high Rep protein expression has been previously shown to inhibit adenoviral replication in *trans,* in a preferred embodiment, the heterologous promoter linked to the *rep* gene is a weak promoter, such as an MMTV promoter, a PGK1 gene promoter, a UBC gene promoter, or an inducible promoter such as a tetracycline-inducible promoter, a Cumate-inducible promoter, an electrosensitive promoter or an optogenetic switch.

In another embodiment, the heterologous promoter may also be a constitutively active promoter such as a herpes simplex virus thymidine kinase (HSV-TK) promoter, a cytomegalovirus (CMV) promoter, a CAG promoter comprising the CMV early enhancer element, the promoter, the first exon and the first intron of chicken beta-actin and the splice acceptor of the rabbit beta globulin gene, a CMV and chicken β-actin (CBA) promoter, a Rous sarcoma virus (RSV) promoter, a human immunodeficiency virus (HIV) promoter, a Simian virus 40 (SV40) promoter, an adenovirus subtype 5 major late promoter (MLP) with or without the first adenovirus subtype 5 intron, a human β-actin promoter, a human elongation factor-1α promoter, an EF1a gene promoter and a human beta actin gene promoter.

In a particularly preferred embodiment, the heterologous promoter of the *rep*-rAdV is a promoter that comprises at least one TATA-Box, at least one SP1-binding site and at least one CCAAT-Box comprising a CTF-binding site. Preferably, the promoter comprises a TATA-Box, two SP1-binding sites (proximal and distal), and a CCAAT-Box comprising a CTF-binding site.

The TATA-Box - also known as Goldberg-Hogness box - is a sequence of DNA commonly found in promoter regions and comprising a consensus sequence characterized by repeating thymine (T) and adenine (A) base pairs. The TATA-Box serves as a transcription factor binding site and is involved in transcription initiation. The Sp1 transcription factor is a so-called zinc finger transcription factor that binds to promoter motifs rich in guanine (G) and cytosine (C) base pairs. A CCAAT-Box is a stretch of DNA comprising the consensus sequence GGCCAATCT and is usually positioned about 60-100 bases to the initial transcription site. The CCAAT-Box serves as transcription factor binding site, such as for the CCAAT box-binding transcription factor (CTF). Preferably, the heterologous promoter is furthermore inducible by the transcription factor E2F, which in turn is inducible by the adenoviral function E1A. E2F-mediated induction is mediated via the above mentioned promoter elements, in particular via the at least one, preferably two Sp1-binding sites. Alternatively, a consensus E2F binding site overlapping with the CCAAT-Box may also be present within the promoter sequence without participating to a major extent in E2F activation.

In case the promoter harbors two Sp1-binding sites, preferably, the distance between the TATA-Box and the upstream first Sp1-binding site located proximally to the TSS (proximal SP-1 binding site) is in the range of 13 - 41 base pairs (bp), e.g., 15-20 bp, 20-25 bp, 25-30 bp, 30-35 bp or 35-41 bp. In a particularly preferred embodiment, the distance between the TATA-Box and the proximal Sp1-binding site is 17-23 bp, e.g., 19 bp.The distance between the proximal Sp1-binding site and the sequence comprising the CCAAT-Box and the second Sp1-binding site located distally from the TSS (distal Sp1-binding site) is preferably in the range of 12-32 bp, e.g., 12-15 bp, 15-20 bp, 20-25 bp or 25-32 bp. Most preferably, the distance between the proximal Sp1-binding site and the region comprising the CCAAT-Box and the distal SP1-binding site is 20-25 bp, e.g., 22 bp. The CCAAT-Box and the distal Sp1-binding site should be closely juxtaposed with a distance of no more than 10 bp between each other. A scheme of an exemplary promoter comprising the mentioned elements in the herein described order is depicted in Fig. 8. The thus described promoter sequences upstream of the TATA Box may also be inverted as long as after inversion the originally distal Sp1-binding site and therefore new proximal Sp1-binding site is distanced about of 13-41 bp away from the TATA box as described above.

In another embodiment, the first, proximal, Sp1-binding site may also be deleted to bring the sequence containing the CCAAT box and the distal Sp1 in close proximity to the TATA box. In such a setting, the element with the CCAAT box and the sole remaining Sp1-binding site may also be in an inverted configuration.

In a particularly preferred embodiment, the promoter is a herpes simplex virus thymidine kinase (HSV-TK) promoter. As shown in the examples below, the inventors found that expression of the Rep proteins under the control of the HSV-TK promoter resulted in a particularly high replication of the rAAV vector genome during rAAV vector production. The HSV-TK promoter is characterized by all of the above described elements and may have a nucleic acid sequence of SEQ ID NO.6. The HSV-TK promoter may also be a variant of a HSV-TK promoter having a nucleic acid sequence of at least 70%, at least 75%, at least 80%, at least 85%, at least 90%, at least 95%, at least 96%, at least 97%, at least 98% or at least 99% sequence identity to SEQ ID NO: 6. The HSV-TK promoter variant may also have nucleic acid sequence having less than 70% sequence identity to SEQ ID NO: 6, e.g. less than 65%, less than 60%, less than 55%, less than 50%, less than 45%, less than 40%, less than 35%, less than 30% or less than 25% sequence identity to SEQ ID NO: 6. In one embodiment, the heterologous promoter operably linked to the 5' end of the rep gene shares only the TATA-Box, one or two SP1-binding sites of 10 bp each and the 12 bp CCAAT box with the HSV-TK promoter of SEQ ID NO: 6.

As indicated above, both the Rep78 and the Rep52 proteins have previously been shown to inhibit adenoviral replication *in trans.* Without being bound by theory, it is believed that this inhibition is mediated by particular amino acids in the carboxy-terminal (C-terminal) part of Rep78 and Rep52 that are encoded by the AAV-2 intron. In particular, the AAV-2 intron encodes the most C-terminal 92 amino acids of both the Rep78 and the Rep52 proteins. These C-terminal, intron-encoded 92 amino acids correspond to amino acids 530 to 621 of Rep78. Of note, also the termination codon for both Rep78/Rep52 is located in said intron. In contrast, the Rep68 and the Rep40 proteins, which arise from alternative splicing of the Rep mRNA, lack these intron-encoded amino acids but instead comprise 7 additional amino acids in their C-terminus encoded by a stretch of nucleotides located immediately downstream of the splice acceptor site. These 7 Rep68/Rep40-specific C-terminal amino acids are in turn not present in Rep78 and Rep52. For some of the other AAV serotypes, e.g., AAV-1, AAV-3, AAV-4, AAV-6, AAV-7, AAV-8, AAV-9, AAV-10, AAV-11 or AAV-12, this applies in a corresponding manner.

It has previously been found that at least some of the 92 C-terminal, intron-encoded amino acids of Rep78/Rep52 mediate direct interaction with the cAMP-dependent protein kinase A (PKA), a kinase which is normally required for adenoviral replication. The interactions between the C-terminus of Rep78/Rep52 and PKA appear to inhibit PKA activity and thus interfere with adenoviral replication. The interaction of Rep78/Rep52 with the PKA was mapped to Rep78 amino acids 526 to 621 (Schmidt, JVirol 2002). In fact, exchange of the specific Rep78 amino acids 533, 536, 539 and 540 appears to be sufficient to disrupt the interactions of Rep78/Rep52 with PKA and thus to abolish the inhibition of adenoviral replication (diPasquale, EMBO J 2003).

Therefore, expression of Rep proteins from a constitutively active promoter such as the HSV-TK promoter or the endogenous AAV-2 p5 promoter is preferably accompanied by a deletion of the AAV intron sequences encoding the C-terminal part of Rep78 and Rep52. For example, the Rep ORF in the recombinant adenoviral rep-vector may be terminated to delete all of the 92 C-terminal amino acids of Rep78 and/or Rep52. The deletion may also encompass less than the 92 C-terminal amino acids of Rep78/Rep52. For instance, the Rep ORF may be terminated to delete only 91 of the intron-encoded C-terminal amino acids of Rep78/Rep52, as described in the examples below. In other embodiments, less than 90, less than 85, less than 80, less than 75, less than 70, less than 65 less than 60, less than 55, less than 50, less than 40, less than 30, less than 20, less than 10 or even less than 5 intron-encoded amino acids are deleted in the truncated Rep78/Rep52 proteins. In some embodiments, it may suffice to delete only selected amino acids in the C-terminus known to be required for mediating the interaction between the Rep78/Rep52 proteins and PKA, e.g., Rep78 amino acids 533, 536, 539 and/or 540. These selected amino acid may also be substituted, i.e., exchanged with other amino acids to abolish interactions of the Rep proteins with PKA.

In a preferred embodiment, the deletion of the intron-encoded amino acids in the Rep78/Rep52 proteins starts almost directly after the splice donor site, by introducing a stop codon at, preferably, nucleotide position 1911 of SEQ ID NO: 1. Insertion of said stop-codon results in a Rep ORF that terminates at amino acid 531 and, therefore, in the expression of a truncated large Rep protein and a truncated small Rep protein. Indeed, as described in the examples below, expression of the thus truncated Rep proteins under the control of the HSV-TK promoter allowed for unexpectedly stable replication of the rep-AdV at high titers of up to 3 x 10¹¹ GP/mL over several rounds of amplification. Advantages associated with deleting the C-terminal part of the Rep proteins as described herein are however not necessarily limited to embodiments where the Rep ORF is linked to a constitutively active promoter such as the HSV-TK promoter. On the contrary, it may be advantageous to express the truncated Rep proteins as herein described, independent of the promoter used for driving Rep expression. Accordingly, in a preferred embodiment, the Rep ORF in the recombinant adenoviral rep-vector is terminated at amino acid 531 by introducing a stop codon at nucleotide position 1911 of SEQ ID NO: 1. It is to be understood that truncating the Rep ORF does not render the recoding of the RIS-Ad dispensable. In contrast, it is the combination of RIS-Ad recoding and truncation of the Rep ORF that is thought to stabilize rep-AdV replication to a particularly high extent. Accordingly, recoding of the RIS-Ad may still initiate at, e.g., nucleotide position 1782 of SEQ ID NO: 1 of the truncated Rep ORF to obtain a nucleic acid sequence corresponding to sRep (SEQ ID NO: 4). The RIS-Ad of the truncated Rep ORF may also be partially recoded as described herein. In any case, recoding preferably ends at nucleotide position 1910 of SEQ ID NO: 1 due to the stop codon being inserted at nt position 1911 of SEQ ID NO: 1.

Recoding of the RIS-Ad and truncation of the Rep ORF at nt position 1911 of SEQ ID NO:1 will result in a *rep*-AdV capable of expressing a large Rep protein, herein referred to as Rep1/530, preferably having an amino acid sequence consisting of the AAV-2 Rep78 amino acids 1-530 according to SEQ ID NO: 5, and a small Rep protein, herein referred to as Rep225/530, preferably having an amino acid sequence consisting of the AAV-2 Rep78 amino acids 225-530 according to SEQ ID NO: 5.

In contrast to Rep1/530, whose expression is preferably under control of a heterologous promoter such as the HSV-TK promoter, the expression of the Rep225/530 protein preferably remains under the control of its physiological p19 promoter. Since the p19 promoter is located within the Rep78/Rep68 ORF, its replacement with a heterologous promoter would otherwise result in an unwanted disruption of Rep1/530 expression.

Suitable first generation rAdVs that may be used in the method of the invention can be prepared by the skilled person. An exemplary *rep*-rAdV comprises a nucleic acid sequence of SEQ ID NO: 7. The rep-rAdV may also comprise a nucleic acid sequence having at least 80%, at least 85%, at least 90%, at least 95%, at least 96%, at least 97%, at least 98%, or at least 99% sequence identity to SEQ ID NO: 7. It may also consist of SEQ ID NO: 7. Alternatively, the first AdV according to the invention may comprise a different promoter region than in SEQ ID NO:7, e.g., another suitable promoter as taught herein.

The second adenoviral vector used for infecting the cell, herein referred to as cap-vector or *cap*-rAdV, comprises an expression cassette comprising the AAV cap gene operably linked to a promoter that is not the wild type p40 promoter.

The AAV cap gene that is to be provided by the *cap*-rAdV encodes the structural proteins VP1, VP2 and VP3 that are assembled into the icosahedral AAV-capsid. In the context of the invention, capsid refers to the protein shell of a virus that encloses the viral genome. Capsids usually consist of repeating structural subunits known as protomers and can be classified according to their geometrical structures.

The capsid proteins encoded by the *cap* gene provided by the *cap*-rAdV may be the capsid proteins of the AAV serotype AAV-2, i.e., the capsid formed upon expression of the *cap* gene in the packaging cell may be an AAV-2 capsid. A viral "serotype" is a variation within single virus species that are classified together based on their surface antigens, i.e., in the case of AAV, based on their serotype-specific viral capsids.

Currently, more than 12 different AAV serotypes and more than 100 naturally occurring capsid variants are known. Serotypes AAV-2, -3, -5, -6 and -9 were first isolated from humans, serotypes AAV-4, -7, -8, -10, -11 and -12 were derived from monkeys and serotypes AAV-1 was isolated both from humans and monkeys. The distinct AAV serotypes may differ substantially in their tissue tropisms, i.e. they may target and infect distinct cell types and tissues. For instance, serotypes AAV-1, AAV-2, AAV-4, AAV-5 and AAV-8 are particularly suitable for transducing cells of the retina, whereas serotypes AAV-6 and -7 may be used for transducing skeletal muscle cells. The capsid of AAV serotype 9 enables AAV to bypass the blood-brain barrier (BBB) and is therefore a leading capsid for the transduction of the central nervous system (CNV) via systemic administration (Wang et al., 2019, Nat Rev Drug Discov.).

Accordingly, the cap gene provided by the *cap*-rAdV may encode the structural capsid proteins of any known AAV serotype or any known capsid variant. In particular, it may encode the capsid proteins of any of AAV serotypes 1, 2, 3, 4, 5, 6, 7, 8, 9 and rh10, wherein, preferably, the structural proteins are from AAV serotype 1, 2, 5, 6, 8 and 9. This allows for production of so-called pseudotyped rAAV-2 vectors that are particularly suitable for transducing a specific cell type or tissue of interest. A person skilled in the art will know how to modify the AAV-2 genome to ultimately obtain a pseudotyped AAV-2 vector. For example, the 5' part of the AAV-2 intron, which is located in a pyrimidine-rich region important for the function of the splice acceptor sites, may be fused with the 3' part of the intron of the respective other AAV-serotype and the subsequent serotype-specific capsid reading frame. Importantly, the inhibition of adenoviral replication by the regulatory sequences for capsid expression localized in the 3' part of the AAV rep gene is not a serotype 2-specific phenomenon, but is also valid for the corresponding genome regions of other AAV serotypes. Thus, it is also necessary to place the capsid proteins of the alternative AAV serotypes under the control of a promoter other than the physiological one.

In another embodiment, the cap gene may encode an AAV capsid that has been specifically modified to improve the targeting specificity of the produced AAV. For instance, the cap gene can be genetically modified to, e.g., express a peptide sequence on the capsid surface that can be bound by specific cellular receptors. An example for such a modification can be found, e.g., in Girod et al. (1999, Nature Medicine 5(9)). Alternatively, peptide sequences such as single-chain variable fragments (scFvs) may be fused, e.g., to the VP2 protein to confer antibody-like recognition of cell surface markers (Wang et al., 2019). In light of the state of the art, a skilled person will be able to think of numerous additional ways for modifying the AAV capsid in order to alter AAV tropism or improve cell-specific vector targeting. The cap gene may also be derived from directed evolution approaches with and without computational-aided pre-selection such as the SCH9 variant, which comprises fragments from AAV serotypes 2, 6, 8 and 9 (Ojala, Molecular Therapy 26, 304-319, 2018)

The cap gene encoded by the cap-AdV may comprise at least one point mutation effective to increase the stability of the AAV capsid and/or change the tropism of the rAAV vector. For instance, site-specific mutagenesis of surface-exposed tyrosine residues Y252, Y272, Y444, Y500, Y700, Y704, and Y730 of the AAV-2 VP3 protein may protect the AAV capsid from proteasomal degradation (Zhong et al., 2008, Proc. Natl. Acad. Sci 105). A study by Wu et al. (2006, J. Virol. 80(22) demonstrated that a lysine residue at position 531 is required for preferential transduction of parenchymal cells by AAV-6. Single point mutation on the T251, T503 site of the AAV-2 or the K137 site of the AAV-8 capsid may dramatically enhance the transduction efficiency of AAV-2 and AAV-8 (Gabriel et al., 2013, Hum Gene Ther Methods 24(2), Sen et al., 2013, Hum Gene Ther Methods 24(2)) The natural tropism of the AAV serotype 2 capsid for heparin sulfate proteoglycans (HSPG) may be ablated by point mutation of arginine residues 585 and 588 to more specifically target the vector to other receptors (Sullivan et al. Gene Therapy 25(3), 205.219, 2018). Therefore, the point mutation may be, preferably, a mutation such as Y444F, Y730F, T251A or R585A/R588A of SEQ ID NO: 14.

As described above, the AAV *cap* gene is normally transcribed from its physiological p40 promoter located in the 3' region of the Rep ORF, which, however, interferes with the replication of the adenoviral vector genome. To overcome this problem, the RIS-Ad within the 3' region of the *rep* gene needs to be recoded, as described herein. However, recoding reduces or completely abrogates the activity of the p40 promoter, thus preventing Cap protein expression if both the *rep* and *cap* genes are delivered in the same AdV. Accordingly, as a first measure, the present inventors decided to split the *rep* and the *cap* genes and provide them from two separate AdVs.

As the wild type p40 promoter constitutes a major element of the RIS-Ad, it cannot be used for generation of rAdVs expressing the AAV Cap proteins. Therefore, as a second measure, the *cap* gene has to be expressed under the control of a promoter that is not the wild type p40 promoter.

In one embodiment, the promoter that is operably liked to the *cap* gene may be a recoded p40 promoter, as described herein. It may thus be a p40 promoter that corresponds in its nucleic acid sequence to the respective area of sRep region described above. The recoded p40 promoter may thus have a nucleic acid sequence having at least 50%, at least 60%, at least 70% at least 75%, at least 80%, at least 85%, at least 90% at least 95%, at least 96%, at least 97%, at least 98% or at least 99% sequence identity to SEQ ID NO: 8. In some embodiments, the recoded p40 promoter may also have a nucleic acid sequence having 100% sequence identity to SEQ ID NO: 8. In some embodiments, the recoded p40 promoter may correspond in its nucleic acid sequence to the wild type p40 promoter with exception to the p40 transcription start site corresponding to nt 1851-1856 of SEQ ID NO: 1 and, optionally, the GGT motif-encompassing p40 region corresponding to nucleotides 1781-1797 of SEQ ID NO: 1, as described above and may have a nucleic acid sequence of SEQ ID NO: 9.

In another embodiment, the promoter that is operably linked to the *cap* gene provided by the *cap*-AdV is a heterologous promoter. The heterologous promoter of the cap-rAdV may be any heterologous promoter suitable for transgene expression in a viral vector. It may, e.g., be a constitutively active promoter such as a CMV promoter, a CAG promoter, a CBA promoter, an RSV promoter, an HIV promoter, an SV40 promoter, an HSV-TK promoter, an MLP with or without the first adenovirus subtype 5 intron, a human β-actin promoter, a human elongation factor-1α promoter, an EF1a gene promoter, a PGK1 gene promoter, a UBC gene promoter and a human beta actin gene promoter. It may also be an inducible promoter as described above such as a tetracycline-inducible promoter, a Cumate-inducible promoter, an electrosensitive promoter and an optogenetic switch However, in a preferred embodiment, the heterologous promoter of the *cap*-rAdV is a promoter that comprises at least one TATA-Box, at least one SP1-binding site, at least one CCAAT-Box and at least one CTF-binding site as described herein.

In a particularly preferred embodiment, the promoter is a HSV-TK promoter or any of the variants thereof described herein.

An exemplary *cap*-rAdV that can be used in the method of the invention comprises a nucleic acid sequence of SEQ ID NO: 10. The *cap*-rAdV may also comprise a nucleic acid sequence having at least 80%, at least 85%, at least 90%, at least 95%, at least 96%, at least 97%, at least 98%, or at least 99% sequence identity to SEQ ID NO: 10. It may also consist of SEQ ID NO: 10. Alternatively, the *cap*-rAdV according to the invention may comprise a different promoter region than in SEQ ID NO: 10, e.g., another suitable promoter as taught herein.

Preferably, both the *rep*-rAdV and the *cap*-rAdV are administered to the cell at an MOI of 100-2000 genomic particles (GP)/cell each, e.g., an MOI of 100-500, 500-1000, 1000-1500 or 1500-2000 GP/cell each. Preferably the two AdVs are administered at an MOI of 150-1000 GP/cell, more preferably at an MOI of 175-750 GP/cell and most preferably at an MOI of 200-400 GP/cell each. The MOI (multiplicity of infection) describes the ratio of viral particles to their infection target, i.e., the target cell that is to be infected. Accordingly, administering an rAdV at an MOI of 200 GP/cell means that a single target cell is inoculated with 200 rAdV particles. The MOI allows indirect conclusions to be drawn about the number of viruses that ultimately enter a given cell. The probability that a cell will absorb a certain amount of virus particles when inoculated with a particular MOI can be calculated for a given cell population using a Poisson distribution. As the MOI increases, the percentages of cells infected with at least one viral particle also increases (https://en.wikipedia.org/wiki/Multiplicity_of_infection)

Moreover, the *rep*-rAdV and the *cap*-rAdV are preferably administered to the cell at a ratio of 1:2 to 2:1, more preferably at a ratio of 1:1.

The cell is furthermore provided with a transgene-vector comprising an expression cassette comprising a transgene operably linked to a promoter, wherein the expression cassette is flanked by AAV inverted terminal repeats (ITR).

The transgene vector preferably is a starter recombinant AAV vector that is provided to the cell alongside the *rep*-rAdV and the *cap*-rAdV. In other embodiments, the transgene vector may however also be a plasmid or a third recombinant adenoviral vector.

In the context of the invention, the term "providing" the transgene vector refers to a method of delivering the transgene vector into the target packaging cell so that the expression cassette comprising the cargo transgene can be packaged into a viral vector particle by said cell. The specific method of transgene vector "provision" depends on the exact nature of the vector. In case the transgene vector is a viral vector such as a starter AAV vector or an adenoviral vector, the transgene vector is provided to the cell via infection of said cell. During infection, the expression cassette comprising the transgene is introduced into the cell by a process known as transduction. Therefore, in case the method of the invention uses a viral vector for transgene delivery, the method is entirely infection-based.

In case the transgene vector is a plasmid, it is preferably provided to the cell via a suitable transfection method known from the state of the art, e.g., via electroporation, microinjection, the use of a gene gun, impalefection, hydrostatic pressure, continuous infusion, sonication, lipofection, non-liposomal reagents such as Fugene or, preferably, calcium phosphate precipitation-based transfection.

In the context of the invention, the terms "transgene", "therapeutic gene" or "cargo sequence" are used synonymously and refer to either a nucleic acid sequence that is effective per se (for example, a regulatory RNA) or a nucleic acid sequence coding for a therapeutic polypeptide or protein, each including all control elements necessary for the expression, such as promoters or post-transcriptional regulatory elements (for example polyadenylation sequences). The choice of the transgene depends on the intended application of the produced rAAV. In case the transgene is a nucleic acid sequence encoding a polypeptide or protein, the transgene preferably is a complementary DNA (cDNA) that has been generated via reverse transcription from messenger RNA (mRNA) transcribed from the gene of interest and that lacks introns normally present in the gene of interest. Alternatively, it may be the complete gene of interest comprising introns and, optionally, regulatory regions provided that the length of the transgene does not exceed the packaging limit of the final rAAV vector. The transgene may alternatively be synthetically engineered. It may, e.g., be codon-optimized to ensure efficient expression of the gene of interest in a target cell, e.g., codon-optimized for expression in human cells. The cargo sequence may, optionally, further comprise an internal ribosomal entry site (IRES), i.e. an RNA element that mediates mRNA translation in the absence of a 5' cap structure and allows for co-expression of multiple genes under the control of a single promoter.

In an alternative embodiment, the cargo sequence may be transcribed into a regulatory RNA selected from the group comprising a microRNA, a short hairpin RNA, a single guide RNA and a long non-coding RNA.

The promoter of the transgene expression cassette is preferably a constitutively active promoter such as a CMV promoter, a CAG promoter, a CBA promoter, an RSV promoter, an HIV promoter, an SV40 promoter, an HSV-TK promoter, a human β-actin promoter, a human elongation factor-1α promoter, an EF1a gene promoter, a PGK1 gene promoter, and a UBC gene promoter, an MLP with or without the first adenovirus subtype 5 intron, an inducible promoter selected from the group comprising a TET-inducible promoter, a Cumate-inducible promoter, an electrosensitive promoter and an optogenetic switch or a tissue specific promoter.

The promoter controlling transgene expression may alternatively also be an AAV-specific promoter. It may, e.g., be an AAV-2 p5, p19 or p40 promoter.

In some embodiments, the expression cassette in the transgene vector may further comprise additional regulatory elements besides the promoter that are capable of enhancing transgene expression. For instance, increasing evidence suggests that many DNA elements that strongly stimulate gene expression are located within introns in the first kilobase of transcribed sequences. Accordingly, the transgene may, e.g., comprise an additional intron capable of enhancing transgene expression. Optionally, the transgene vector may further comprise a woodchuck hepatitis post-transcriptional regulatory element (WPRE). The WPRE RNA sequence creates a tertiary structure enhancing expression when transcribed from the DNA. The sequence is commonly used in molecular biology to increase expression of genes delivered by viral vectors. The transgene is furthermore preferably codon-optimized, i.e. particular codons in the transgene may be substituted by synonymous codons that are preferably used by an organism such as a human to improve gene expression. Codon optimization may help to overcome the impact of a disfavorable GC content, cryptic splice sites, transcription termination signals, motifs that affect RNA stability and/or nucleic acid secondary structures that may be normally present in the transgene.

The terminal AAV ITRs that flank the expression cassette of the transgene vector are required at the nucleic acid level (*in cis*) for genome replication and packaging of the replicated AAV genome. Preferably, the AAV ITRs are derived from the AAV-2 serotype. In alternative embodiments, the AAV ITRs may also be derived from an AAV serotype other than serotype 2.

As described above, the transgene vector preferably is a "starter rAAV vector". A starter rAAV is a viral rAAV vector used to initiate rAAV production. It is identical to the rAAV vector that is ultimately for use in clinical research or gene therapy. A starter rAAV vector is pre-produced in relatively small amounts and is subsequently amplified by the method of the invention over several rounds to obtain vector titers sufficiently high for pre-clinical and clinical applications. The present inventors found that they were able to amplify a starter rAAV by a factor of up to 1000 to 1500-fold using the method of the invention. The starter rAAV vector may have been originally obtained by any rAAV vector production method known to the skilled person. For example, the starter rAAV vector may be obtainable, e.g., by traditional co-transfection of a packaging cell with a mixture of a vector plasmid and one or more helper plasmids, as described above. Alternatively, the starter rAAV vector may be obtainable via an infection-based production method, such as by infecting, e.g., an insect cell with baculoviruses encoding an AAV-ITR flanked transgene cassette of interest and the AAV Rep and Cap proteins. The starter rAAV vector may also be obtained by the method of the invention, i.e., a rAAV vector that was obtained through the herein disclosed method may serve as a new starter rAAV vector for an ensuing round of rAAV vector amplification. Accordingly, the method of the invention can be repeated for several rounds for continuous rAAV vector amplification to maximize the final vector yield. The starter rAAV vector can be administered to the cell at relatively low MOIs in the range of 10-1500 GP/cell, e.g., at a MOI of 10-100, 100-500, 500-1200 GP/cell or, preferably, at a MOI of 20-50 GP/cell or less.

An exemplary starter rAAV vector lacking a transgene that can be used as a transgene vector in the method of the invention comprises a nucleic acid sequence of SEQ ID NO: 11. The starter rAAV vector may also comprise a nucleic acid sequence having at least 80%, at least 85%, at least 90%, at least 95%, at least 96%, at least 97%, at least 98%, or at least 99% sequence identity to SEQ ID NO: 11. Alternatively, the starter rAAV vector according to the invention may comprise a different promoter region than in SEQ ID NO: 11, e.g., another suitable promoter as taught herein.

In another embodiment, the transgene vector may be starter recombinant adenoviral vector. A starter recombinant adenoviral vector is a recombinant adenoviral vector vector used to initiate rAAV production. Thus, the starter recombinant adenoviral vector is a third recombinant adenoviral vector that is administered to the infected cell alongside with the rep-rAdV and the cap-rAdV. Dependent on the size of the transgene, the transgene-AdV may be a first, a second or a third generation AdV. Like the rep-rAdV and the cap-AdV, the transgene AdV may be administered to the cell at a MOI of 100-500, 500-1000, 1000-1500 or 1500-2000 GP/cell. Preferably it is administered at a MOI of 150-1000 GP/cell, more preferably at a MOI of 175-750 GP/cell and most preferably at a MOI of 200-400 GP/cell.

In other embodiments, the AVV-ITR transgene-comprising expression cassette may also be provided by a viral vector that is not a starter AAV or an AdV. For instance, the viral transgene vector may be derived from a retroviral vector. It may, e.g., be a lentiviral vector.

The transgene vector may also be a starter transfection plasmid, i.e., a plasmid used to initiate rAAV production. Plasmids are relatively small circular DNA molecules that are widely used as vectors in molecular cloning. In case the transgene vector is a plasmid, said plasmid may be a plasmid that is derived from either bacteria or yeast. A person skilled in the art will be able to choose from various plasmid types known from the state of the art to identify a suitable plasmid for transgene delivery. A suitable transgene plasmid for AAV vector production is, e.g., the pTR-UF5 plasmid, as described in the examples below.

When the method of the invention is for producing AAV vectors rather than empty virus-like particles, the transgene vector preferably is a starter rAAV vector. The inventors found that provision of the transgene via a plasmid vector or a third rAdV resulted in higher levels of empty capsids. Some reports suggest that empty capsids may serve as decoys for antibodies produced by the immune system against AAV vectors, and may thus help to prevent antibodies from capturing co-administered rAAV particles carrying a transgene. However, a high fraction of empty AAV capsids obtained during AAV vector production is usually less desired. Empty capsid can further stimulate unwanted immune responses against the AAV vectors and thus reduce the chances of success of a given gene therapy.

As described herein, the cell that is infected with the *rep-* and the *cap*-rAdVs and provided with the transgene vector serves as a packaging cell for rAAV vector assembly. The cell is preferably a cell of a human cell line, such as, e.g., a human embryonic kidney 293 (HEK293) cell, a HEK293T cell, or a HeLa cell. Preferably, the cell is a HEK293 cell or a HEK293T cell, most preferably a HEK293 cell. The cell may be an adherent cell, i.e. it may require a surface or an artificial substrate for growth. However, the production method according to the invention can be easily adapted to vector production in free floating cells, i.e. suspended cells, thus allowing for larger scale AAV vector production. Therefore, the cell used as a packaging cell preferably is a cell in suspension that is present in a suitable medium, e.g. a serum-free medium such as Gibco^{®} CD 293 Medium by ThermoFisher Scientific. Accordingly, the cell may be, e.g., a Gibco^{®} 293-F cell. The suspended cell may be infected with the *rep*-AdV, the *cap-*AdV and, optionally, the transgene rAdV at an MOI of about 100-2000 GP/cell, respectively, e.g., an MOI of 100-500, 500-1000, 1000-1500 or 1500-2000 GP/cell, respectively. In case the transgene vector is a starter rAAV, it may be administered to the suspended cells at an MOI of 10-1500 GP/cell, e.g., at a MOI of 10-100, 100-500 or 500-1200 GP/cell. Infection of the suspended cells may last for 24-96h, e.g. for 24-36 h, 36-48 h, 48-60 h, 60-72 h, 72-84 h or 84-96 h. Preferably, infection of the suspended cells lasts for 64-96 h.

Due to the provision of the *rep* and the *cap* genes via rAdVs, the cell furthermore does not need to be genetically modified to provide the adenoviral helper functions.

In case that the transgene vector is a viral vector, the two rAdVs carrying the *rep* and the *cap* genes as well as the transgene vector can be administered simultaneously to the cell via co-infection. Alternatively, the vectors may be administered sequentially, e.g., the viral transgene vector is administered first, followed by provision of the two rAdVs comprising the *rep* and *cap* genes. The order of infection is deemed to be of no particular relevance. In case the transgene vector is a plasmid, the cell may first be infected with the two rAdVs before being transfected with the transgene vector plasmid, or vice versa.

After infecting the packaging cells with the different AdVs and providing the transgene vector, the newly generated viral particles need to be harvested. The right time point for virus harvesting is characterized by structural changes in the packaging cells caused by the viral invasion. These structural changes are summarized by the term "cytopathic effect", which is well known to a person skilled in the art. The time between infection/transfection of the package cell and harvesting of the produced AAV vector particles or the AAV virus-like particles may take several hours to several days, e.g., 12-96 h, 24-72 h, 36-60 h, or 40-50 h. The present inventors found that viral particles could be usually harvested about 64-72 h after infection/transfection of the packaging cells.

As a typically final step of the method of the invention, the newly produced and harvested rAAV vectors or AAV virus-like particles are purified according to standard laboratory technologies known to a person skilled in the art. AAV particle purification may for instance be achieved via chromatographical methods, e.g., an HPLC affinity chromatography with AVB sepharose as described in the examples below. The rAAV vectors or virus-like particles may further be formulated and or packaged for administration.

Further disclosed by the present invention is a method specifically for producing empty AAV virus-like particles (AAV-VLPs) comprising infecting the cell with the recombinant adenoviral *cap*-vector described herein. The inventors found that infection of HEK293 cells alone with the *cap*-AdV was sufficient to produce high levels of empty AAV capsids. These could be purified in high yield by affinity chromatography in the same way as complete rAAV vectors. The AAV-VLPs can be for use in, e.g., overcoming an existing B-cell immune response against AAV, as they act as binding dummies for existing antibodies. Other possible applications of AAV-VLPs are, e.g., in diagnostics, where they can be used as antigens for the specific detection of antibodies against different AAV serotypes.

Finally, the present invention is further directed to a kit or a composition for producing a recombinant adeno-associated virus (rAAV) vector according to the method of the invention, wherein said kit or composition comprises
a) a transgene-vector comprising an expression cassette comprising a transgene operably linked to a promoter, wherein the expression cassette is flanked by AAV inverted terminal repeats (ITR),
b) a recombinant adenoviral *rep*-vector (*rep*-rAdV) comprising an expression cassette comprising the AAV *rep* gene operably linked to a promoter, wherein the rep gene does not comprise a RIS-Ad and wherein the *rep-*vector does not encode the AAV Cap proteins, and
c) a recombinant adenoviral *cap*-vector (*cap-*AdV) comprising an expression cassette comprising the AAV cap gene operably linked to a promoter that is not the wild-type p40 promoter, wherein said *cap*-vector does not encode the AAV Rep proteins.

The transgene vector preferably is a starter rAAV vector comprising an expression cassette comprising a transgene operably linked to a heterologous promoter, wherein the expression cassette is flanked by AAV inverted terminal repeats (ITR).

The kit may comprise a single compartment in which the three different viral vectors are mixed together. Alternatively, the three different viral vectors are physically separated into up to three distinct compartments that are only mixed prior to or during use of the kit. The kit may also comprise two compartments, wherein, preferably, one compartment comprises the starter rAAV vector and the other compartment comprises the two AdVs. The components of the kit, i.e. the three different viral vectors may also not be mixed at all, e.g., they may be administered to a suitable packaging cell separately during three separate infection steps. The different vectors may be present in a suitable storage medium such as PEG 6000 or a solution comprising glycerol to stabilize the viral vectors during freezing. The kit or composition may be stored at temperatures of 2-6°C for several days to several weeks, e.g., 1 day to 4 weeks, 1 day to 3 weeks, 1 day to 2 weeks or 1 day to 7 days. Preferably, the kit or composition may be stored at 2-6°C for less than 2 weeks. For long term storage, the kit or composition may be stored at -80°C for a period between several weeks to several month before use, e.g. from 4 weeks to 24 months, from 2 months to 20 months, from 4 months to 16 months, from 8 months to 14 months of for about 12 months. In some embodiments, the kit or composition may be stored at -80°C for even longer periods before use, e.g. for 3 years, for 4 years, for 5 years, for 6 years, for 7 years, for 8 years, for 9 years, or even for up to 10 years.

The herein disclosed manufacturing method and kits for AAV vector production allow, e.g., small quantities of pre-produced rAAV to be amplified by a factor of up to 1000 to 1500-fold in a single simple co-infection step. The yields per initially inserted cell are in the range of 2-4 x 10⁴ genomic copies/inserted cell after affinity chromatic purification of the newly produced rAAVs. Furthermore, the time-consuming generation of new transgene-specific packaging cell lines or helper viruses, as is absolutely necessary with other infection-based rAAV packaging methods, is not required. Due to the simple handling and the high rAAV yields, this novel production system should be of interest to all users who e.g. want to quickly bring an rAAV vector already characterized in the laboratory into testing in the animal model and into clinical application.

Throughout the invention, the term "about" is intended to be understood as "+/- 10%". If "about" relates to a range, it refers to both lower and upper limit of the range. "A" is intended to mean "one or more", if not explicitly mentioned otherwise.

All literature cited herein is herewith fully incorporated. The present invention is further illustrated, but not limited, by the following examples.

### Figure Legends

**Fig. 1** **Infection cycle of the adeno-associated virus (AAV).** Bipartite infection cycle of AAV serotype 2 in cell culture with productive infection in the presence of a helpervirus such as adenovirus or herpes simplex virus (HSV) and latent infection with partially site-specific integration onto AAV-S1 site on human chromosome 19 in the absence of helpervirus. From the latent state, AAV-2 can be rescued by infection with a helper virus and enter a productive replication cycle again.
**Fig. 2** **Genomic organization of the AAV serotype 2.** Genome organization of AAV-2 with the inverted terminal repeats (ITRs), the four Rep proteins Rep78, Rep68, Rep52, Rep40, the capsid proteins VP1 to VP3 and the AAP (*assembly activating* protein) indicated by boxes. The corresponding transcripts are indicated by arrow-headed lines. Right-angled arrows represent the transcription start sites of the three AAV-2 promoters at map units 5, 19 and 40 and the vertical arrow indicates the common polyadenylation (polyA) site for all transcripts at map position 96.

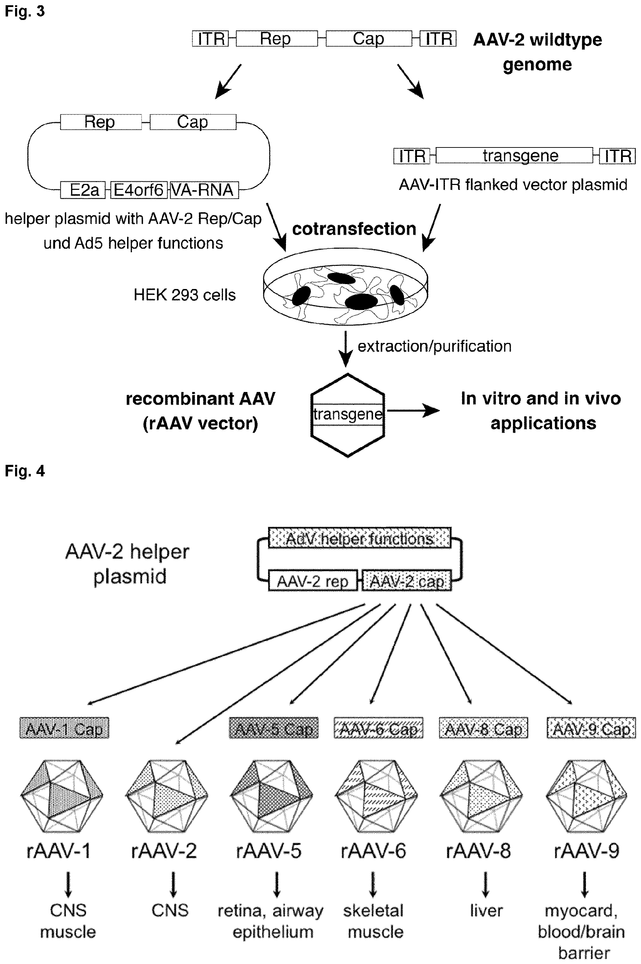

**Fig. 3** **Standard laboratory method for the production of recombinant AAV vectors (rAAV).** Standard two-plasmid co-transfection system for the production of rAAV with the helper plasmid (such as the pDG plasmid) encoding the AAV-2 Rep and Cap Proteins and the adenovirus type 5 (Ad5) E2a, E4orf6 and VA-RNA functions, and the vector plasmid harboring an AAV-2 ITR-flanked transgene cassette. The additionally required Ad5 E1a and E1b functions are provided by the HEK293 cells
**Fig. 4** **Helper plasmids for the production of pseudotyped AAV vectors.** Exchange of the AAV-2 capsid gene in the standard helper plasmid for the capsid genes of AAV-2 serotypes 1, 5, 6, 8 and 9. These altered cap genes and the corresponding assembled capsids are indicated by differentially patterned boxes. The preferred target organs of the pseudotyped rAAV vectors are shown below.
**Fig. 5** **Production of recombinant AAV vectors using recombinant HSV/AAV- hybrid vectors.** Packaging of rAAV vectors with a recombinant HSV (rHSV) vector providing the rep and cap functions (left side) and a second rHSV vector providing the AAV-ITR flanked transgene (right side).
**Fig. 6** **Production of recombinant AAV vectors using recombinant adenoviral/AAV hybrid vectors.** Possible packaging of rAAV vectors with a rAd vector (rAdV) providing the rep and cap functions (left side) and a second rAdV providing the AAV-ITR flanked transgene.
**Fig. 7** **Probable mechanism of inhibition of adenoviral replication by AAV-2 nucleotides 1701-2186 (RIS-Ad).** (A) Elements in the AAV-2 p40 promoter possibly involved in RNA Pol II pausing. The combination of the Inr element, the pause button (PB) and the GAGA element has been shown to be highly predictive for promoters with disproportionately high levels of Pol II near the TSS. Nucleotide positions are indicated relative to the TSS. Abbreviations: TBP, TATA box binding protein; GAF, GAGA factor. (B) Proposed model of inhibition of Ad5/AAV hybrid vector genome replication through steric hindrance by a stalled RNA Pol II complex at the p40 promoter. Abbreviations: DSIF, DRB sensitivity-inducing factor; NELF, negative elongation factor.
**Fig. 8** **Scheme of exemplary promoters for the expression of the large Rep genes.** (A) Promoter comprising a transcription start site (TSS), a TATA-Box, a proximal and a distal Sp1-bining site and a CCAAT-Box (CTF-binding site). (B) and (C) Alternative promoters with only one Sp1 site. The preferred distances between the different elements are indicated in base pairs (bp)

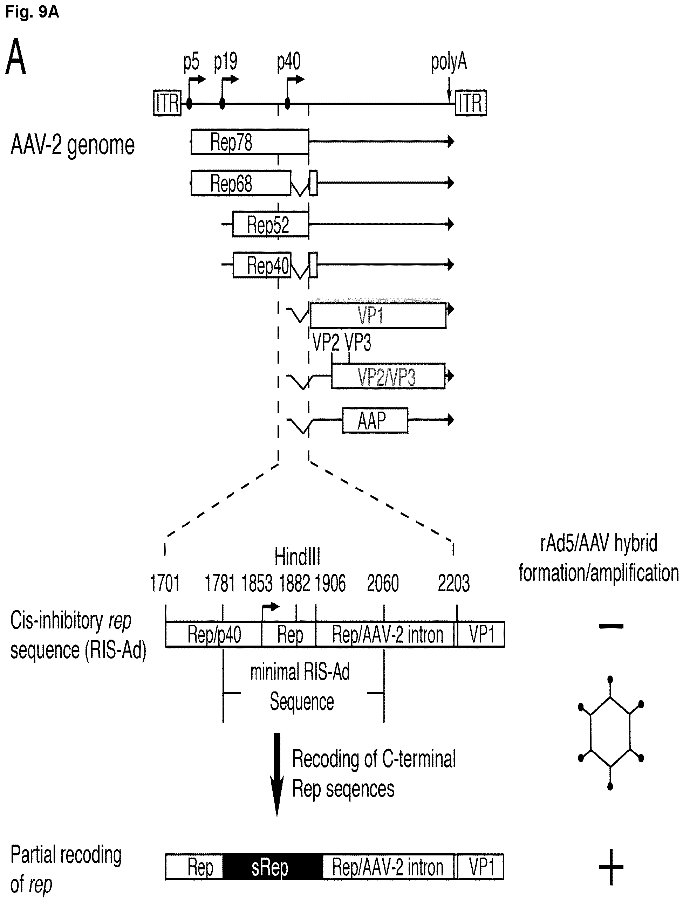

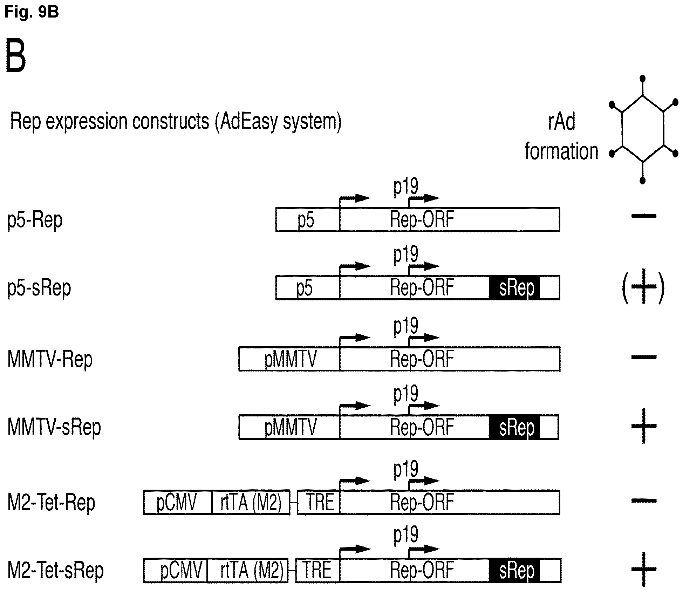

**Fig. 9** **Expression of AAV-2 Rep proteins by adenoviral shuttle plasmids after recoding of the 3'-inhibitory RIS-Ad sequence.** (A) Genome organization of AAV-2 with a close-up of the RIS-Ad (*Rep inhibitory sequence for adenoviral replication)* in the 3'-part of the *rep* gene. The AAV-2 inverted terminal repeats (ITRs), the four Rep proteins Rep78, Rep68, Rep52, Rep40, the capsid proteins VP1 to VP3 and the AAP (*assembly activating* protein) are indicated by different boxes. Right-angled arrows represent the transcription start sites of the three AAV-2 promoters at map units 5, 19 and 40 and the vertical arrow indicates the common polyadenylation (polyA) site for all transcripts at map position 96. The close-up of the extended RIS-Ad region and the recoding of AAV-2 nucleotides 1782 to 1916 (sRep) sufficient for abolishing the inhibitory function of the RIS-Ad are shown in the lower part of the figure. Characteristic nucleotide positions are given above the boxes. (B) Schematic presentation of the transgene cassettes of the shuttle plasmids used for rAd generation (pShuttle constructs for homologous recombination in *E.coli* and the resulting pAdEasy plasmids for transfection of HEK293 cells). Boxes represent the Rep78/Rep52 ORF, the recoded (sRep) sequence and the promoter cassettes. Formation of rAdV after transfection of the corresponding Pacl-linearized pAdEasy plasmids into HEK293 cells is indicated by "+" and "- " signs. "(+)" indicates an unstable rAdV.

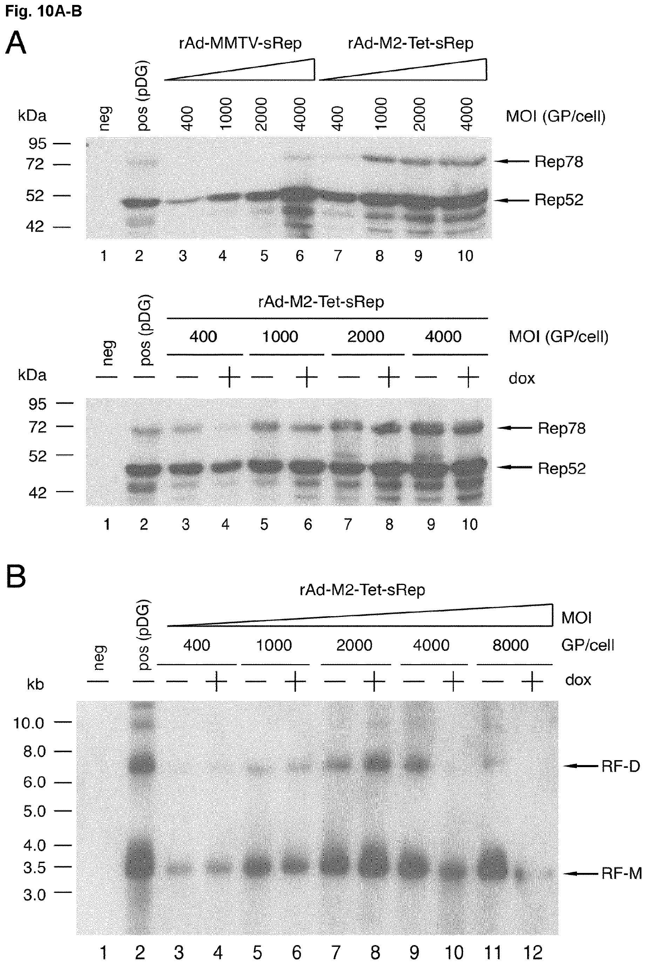

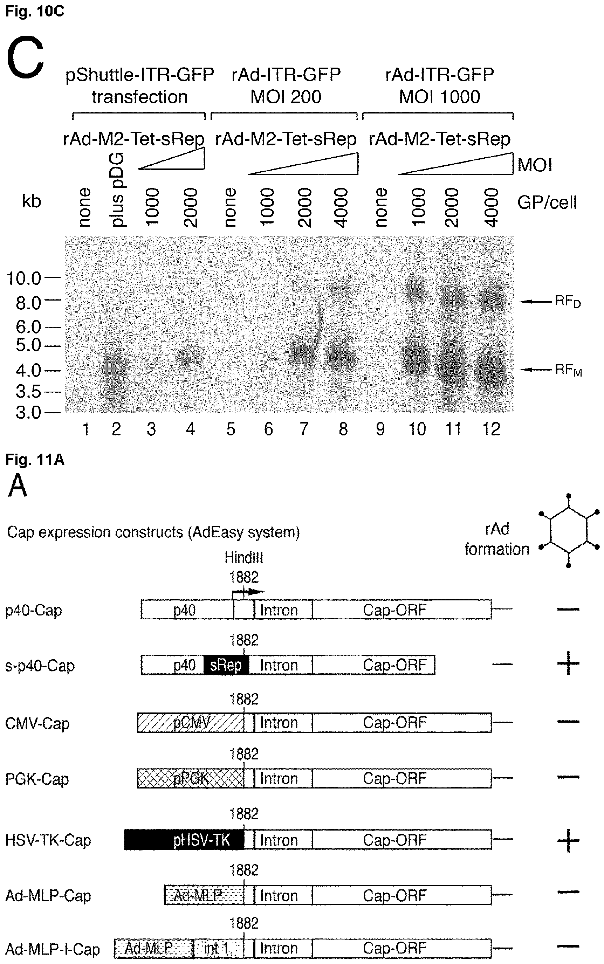

**Fig. 10** **Charaterization of rAdVs expressing functional Rep proteins.** (A) Anti-Rep western blot analysis after infection of HEK293 cells for 64 h with rAd-MMTV-sRep (amplification round 5) and rAd-M2-Tet-sRep (round 6) at the indicated MOIs (genomic particles/cell). Transfection of pTR-UF5 rAAV vector plasmid was used as negative (lane "neg") and pDG plasmid transfection (transfection efficiency about 70%) as positive control. The upper panel shows a comparison of rAd-MMTV-sRep and rAd-M2-Tet-sRep (the later in the presence of doxycycline) at equal MOIs, whereas the lower panel shows the effect of doxycycline (1 µg/ml final concentration, denoted as "dox") with rAd-M2-Tet-sRep at different MOIs. (B) and (C) Southern blot analysis for rAAV replicative DNA intermediates. Viral DNA was extracted 64 hours after transfection/infection and hybridized with a probe for the respective transgene cassette. Arrows indicate monomeric and dimeric replicative rAAV-DNA intermediates (denoted as RF-M and RF-D). (B) An AAV-2 ITR flanked CMV-GFP transgene cassette was provided by transfection of HEK293 cells with pTR-UF5 and cells were additionally infected with rAd-M2-Tet-sRep in the absence or presence of doxycycline at the indicated MOIs for 64 h. The positive control in lane 2 represents pTR-UF5/pDG co-transfected cells. (C) An AAV-2 ITR flanked CBA-GFP cassette was provided either in form of a plasmid (pShuttle-ITR-GFP) or by infection with the corresponding rAd (rAd-ITR-GFP) at two different MOIs as indicated. Lane 2 represents pShuttle-ITR-GFP/pDG co-transfection.

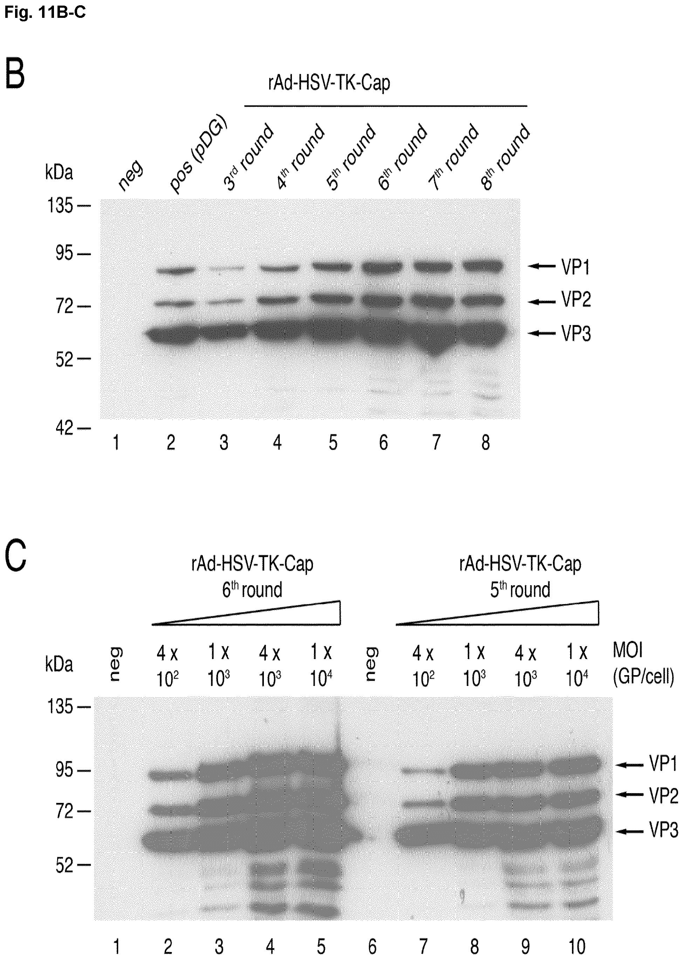

**Fig. 11** **Generation of rAdVs expressing the AAV-2 capsid proteins under control of the herpes simplex virus thymidine kinase (HSV-TK) promoter.** (A) Schematic presentation of the adenoviral shuttle plasmids for AAV-2 Cap protein expression. Different boxes denote the Cap ORF, the major AAV-2 intron, the recoded (sRep) sequence and the different promoter cassettes. Formation of rAdV after transfection of the corresponding Pacl-linearized pAdEasy plasmids into HEK293 cells is indicated by "+" and "-" signs. (B) Infection of HEK293 cells for 64 h with equal amounts of freeze-thaw supernatants from amplification rounds 3 to 8 of rAd-HSV-TK-Cap amplification with subsequent anti-Cap western analysis of whole cell extracts. Arrows indicate positions of capsid proteins VP1 to VP3. pDG transfected HEK293 cells were used as positive control. (C) Anti-Cap western blot analysis after infection of HEK293 cells with rAd-HSV-TK-Cap freeze-thaw supernatants from amplification rounds 6 (left lanes) and 5 (right lanes) at the indicated MOIs for 64 h.

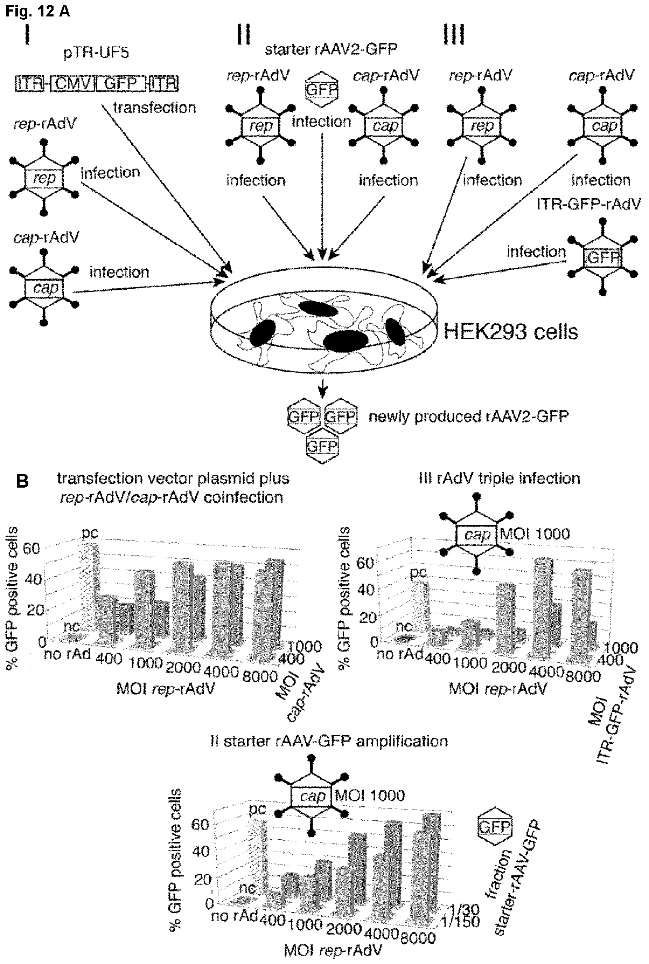

**Fig. 12** **Optimization of rAdV-mediated rAAV2-GFP vector packaging. A** The upper part of the figure outlines the different experimental approaches for rAdV-mediated packaging of rAAV2-GFP vectors in HEK293 cells: (I) infection of pTR-UF5 transfected cells with a mixture of rAd-M2-Tet-sRep (denoted as rep-*rAdV*) and rAd-HSV-TK-Cap (denoted as cap-rAdV), (II) amplification of low input amounts of starter rAAV2-GFP by coinfection with rep-rAdV and cap-rAdV and (III) triple rAd infection with ITR-GFP-rAdV, rep-*rAdV* and cap-rAdV. **B** The lower part of the figure shows the percentage of GFP positive cells obtained after transduction of 3 x 10⁵ HeLa cells with small aliquots (1/15) of freeze-thaw lysates from packaging experiments performed with 5 x 10⁵ HEK293 cells in 6-well plates. The denoted MOIs in GP/cell refer to those used for the packaging in HEK293 cells. In (II) and (III), rAd-Cap was used at a MOI of 1000 GP/cell. Positive controls were performed by means of the standard co-transfection protocol with pTR-UF5 and pDG. For the rAdV-mediated rAAV-GFP amplification (II), 1/30 or 1/150 fractions, as indicated, from the freeze-thaw lysate of a 6-well positive control were used as starter rAAV-GFP for the next-step rAdV-mediated amplification in the same 6-well format.

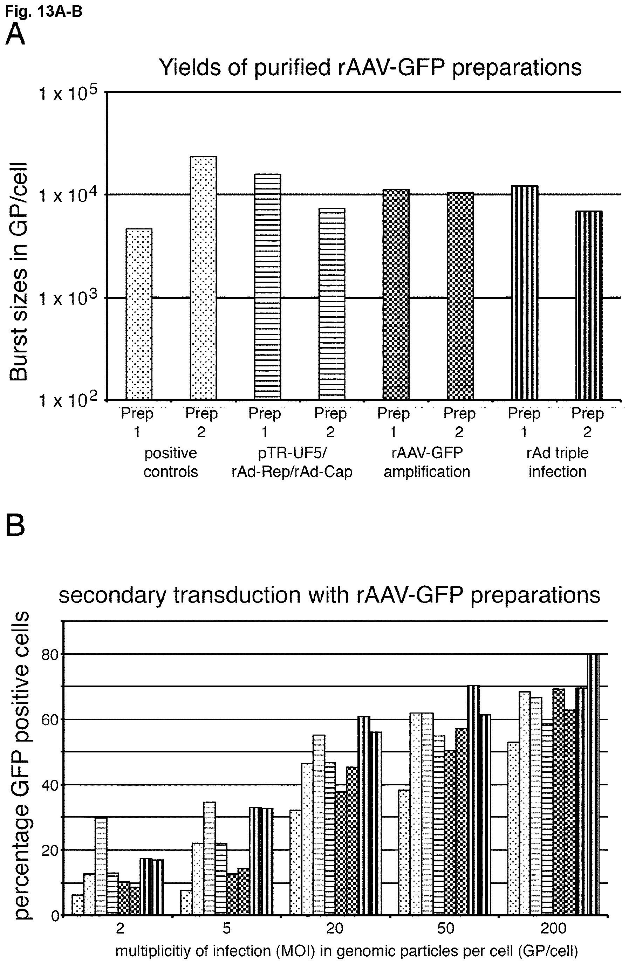

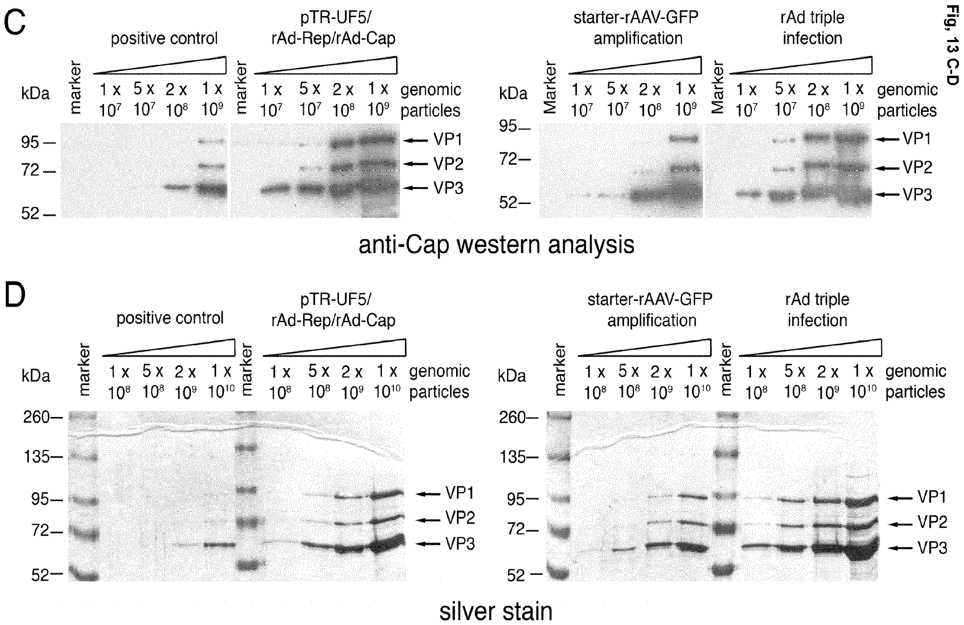

**Fig. 13** **Characterization of affinity-purified rAAV2-GFP vector preparations generated with the help of recombinant adenoviruses.** (A) Burst sizes in genomic particles per cell (GP/cell) for individual affinity-purified rAAV2-GFP vector preparations generated with different packaging approaches as indicated. The positive controls correspond to the standard cotransfection protocol, the rAdV-mediated packaging protocols have been shown in detail in Fig. 12. Two independent, representative preparations are shown for each setup. (B) Percentage of GFP positive cells after transduction of HeLa cells with 5 different MOIs (expressed in GP/cell) of the affinity purified rAAV2-GFP preparations, with the pattern coding corresponding to the different setups denoted in (A). (C) Western blot analysis of capsid protein content with four different amounts of genomic particles (1 x 10⁷, 5 x 10⁷, 2 x 10⁸ and 1 x 10⁹) from the purified rAAV2-GFP vector preparations shown in (A). The respective preparation with the higher burst size was chosen for analysis for each of the four different packaging methods. (D) Silver staining of SDS PAGE gels loaded with different amounts of genomic particles (1 x 10⁸, 5 x 10⁸, 2 x 10⁹ and 1 x 10¹⁰) from the same rAAV2-GFP vector preparations as in (C).

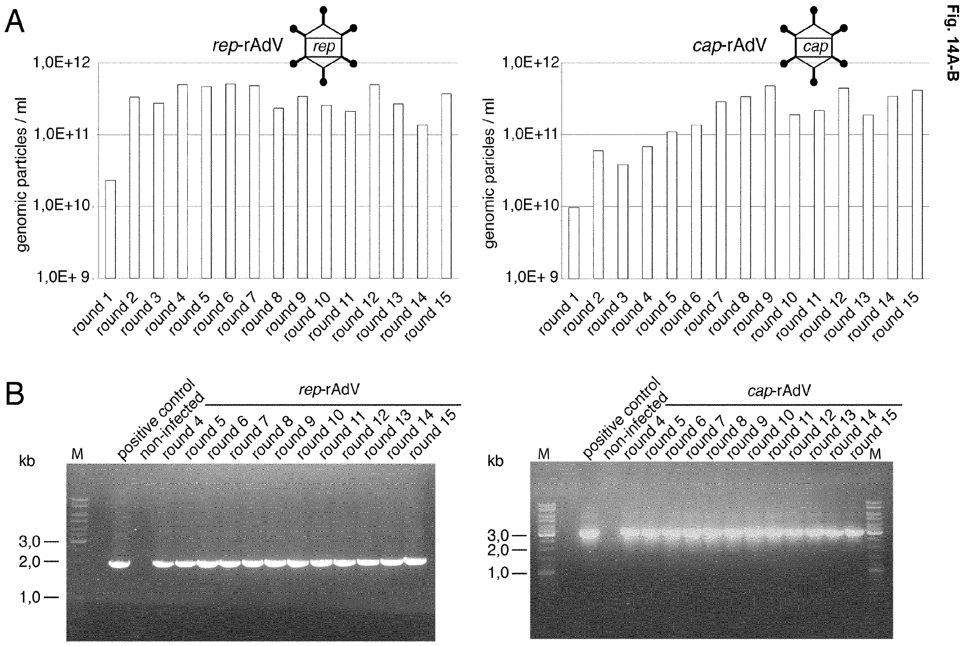

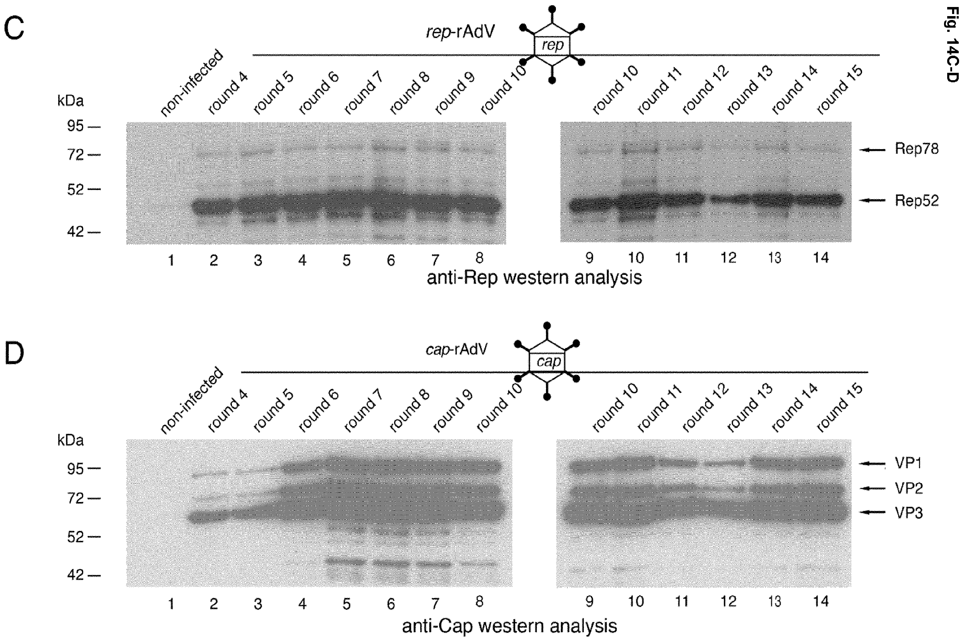

**Fig. 14** **Yield, genomic stability and protein expression of rAd-M2-Tet-sRep and rAd-HSV-TK-Cap over serial amplification rounds.** rAd-Me-Tet-sRep (*rep*-rAdV, left panels in A and B) and rAd-HSV-TK-Cap (cap-*rAdV*, right panels in A and B) were amplified in HEK293 cells over 15 rounds. Essentially, 1 x 10⁷ cells (175 cm² flasks) were infected with 2.5 x 10¹⁰ genomic rAd particles from the previous round for 44 to 50 h and lysed in 5 ml of residual medium by three freeze-thaw cycles. (A) Genomic rAdV titers in the freeze-thaw supernatants determined by PCR-analysis as described in the method section. (B) Analysis of the genomic integrity by PCR-amplification with oligonucleotides binding at the very ends of the respective transgene cassette. The pAdEasy plasmids used for generation of the rAdVs were used as positive controls. (C) and (D) Anti-Rep (C) or anti-Cap (D) western blot analysis after infection of HEK293 cells with freeze-thaw supernatants from the indicated rep-rAdV or cap-*rAdV* amplification rounds at a MOI of 200 for 64 h.

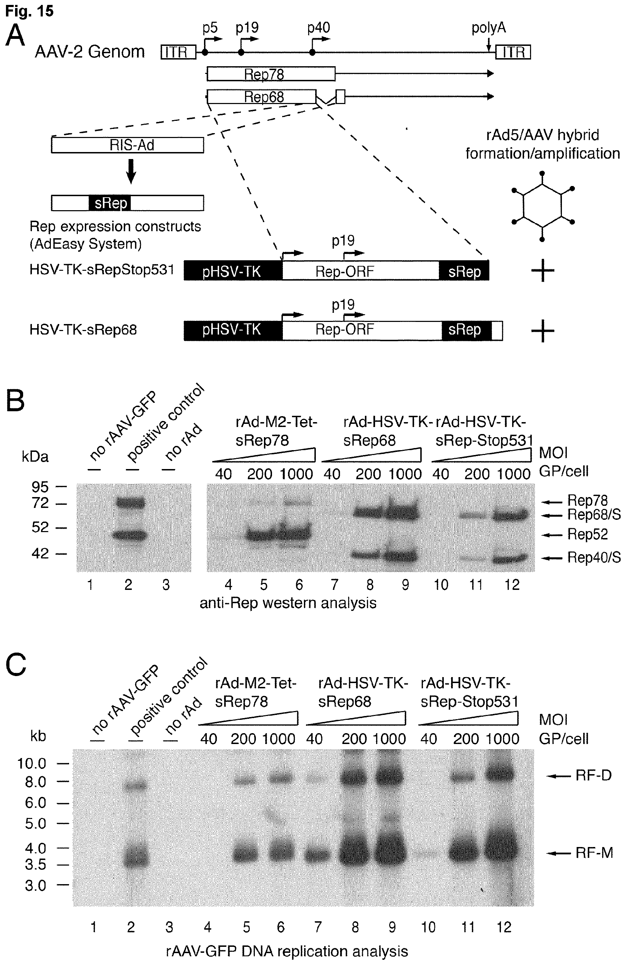

**Fig. 15** **Improved rAd-mediated expression of functional large Rep proteins by means of the constitutive HSV-TK promoter.** (A) Genome organization of AAV-2 with a close-up of the RIS-Ad (*Rep inhibitory sequence for adenoviral replication*) in the 3'-part of the rep gene (for further details compare fig. 9A) and depiction of the large Rep protein Rep68 and the truncated sRep1/530 protein, which terminates immediately after the splice donor site. Both are recoded in the region from AAV-2 nucleotides 1782 to 1906. Different boxes represent the Rep-ORF, the recoded region (sRep) and the HSV-TK promoter used for expression of the proteins in the adenoviral shuttle plasmids. (B) Anti-Rep western blot analysis after co-infection of HEK293 cells with purified starter rAAV-GFP (MOI 100) and either rAd-M2-Tet-sRep, rAd-HSV-TK-sRep68 or rAd-HSV-TK-sRep1/530 as indicated, for 64 h. MOIs in genomic particles/cell are also indicated. Cells co-transfected with pTR-UF5 and pDG were used as positive control. (C) Identical experimental approaches as in (B), but viral DNA was extracted 64 hours after transfection/infection and hybridized with a probe for the CMV-GFP transgene cassette. Arrows indicate monomeric (RF-M) and dimeric replicative (RF-D) rAAV-GFP DNA intermediates.

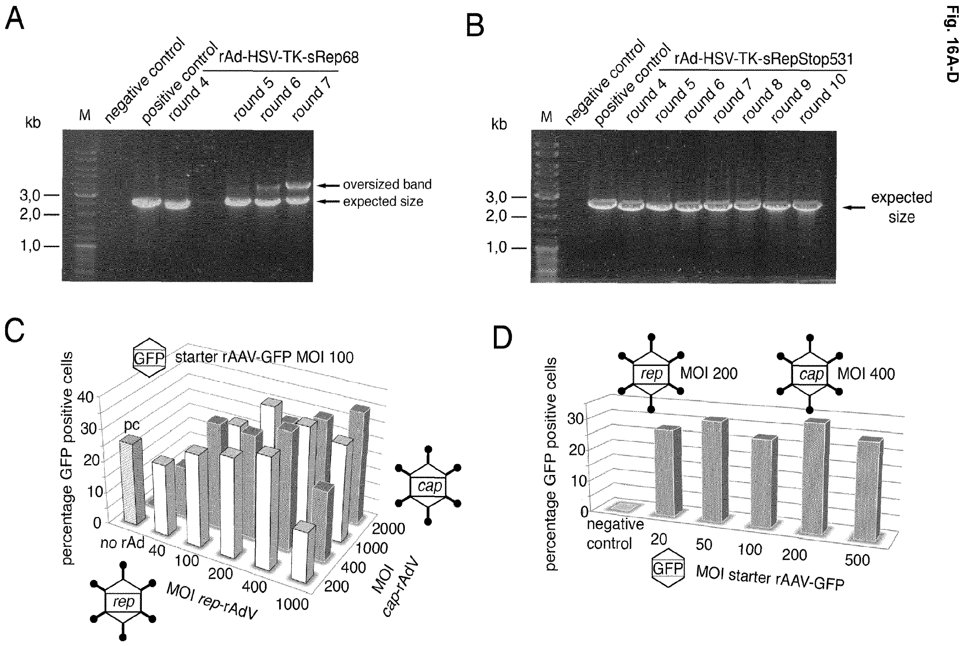

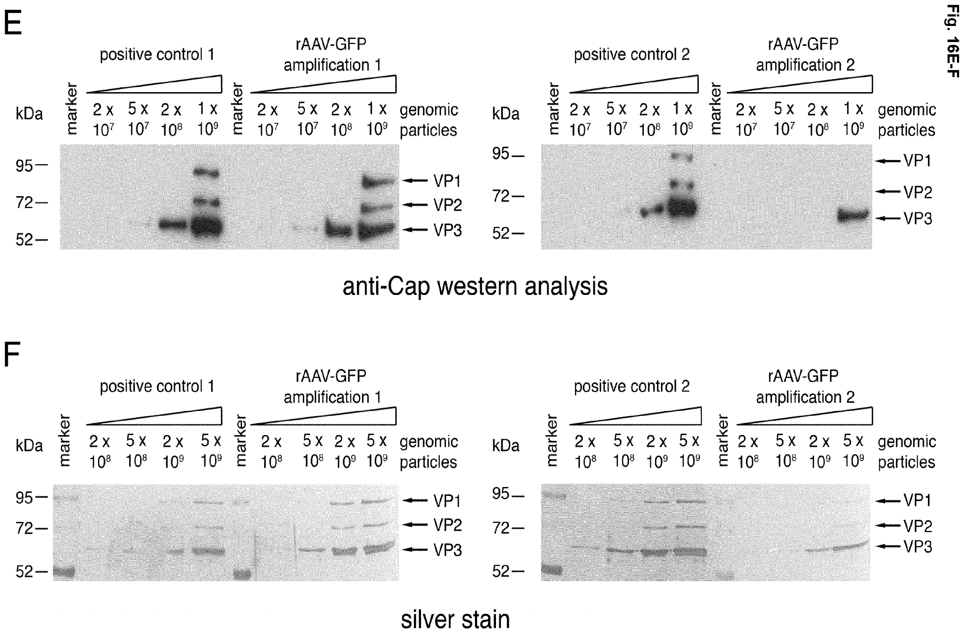

**Fig. 16** **rAAV-GFP amplification after rAdV-mediated expression of truncated Rep proteins under HSV-TK promoter control.** (A) and (B) Analysis of the rAdV transgene cassette in supernatants from different amplification rounds of (A) rAd-HSV-TK-sRep68 and (B) rAd-HSV-TK-sRep1/530. The oligonucleotides used for PCR amplification bind within the adenoviral sequences flanking the transgene cassette in the pAdEasy plasmids. These pAdEasy plasmids used for generation of the rAdVs were also used as positive controls for the PCR amplification. The PCR-products of the expected sizes and an oversized band observed in probes from higher amplification rounds of rAd-HSV-TK-sRep68 are indicated by arrows. (C) and (D) Percentage of GFP positive cells obtained after transduction of 3 x 10⁵ HeLa cells with small aliquots (1/30) of freeze-thaw lysates from rAd-mediated amplification of rAAV2-GFP performed with 5 x 10⁵ HEK293 cells (6-well format). (C) Constant amounts for the rAAV-GFP input vector (MOI 100) and varying MOIs of rAd-HSV-TK-sRep1/530 (denoted as *rep-*rAdV) and rAd-HSV-TK-Cap (denoted as cap-rAdV) as indicated were used. (D) Constant amounts of rAd-HSV-TK-sRep1/530 (MOI 200) and rAd-HSV-TK-Cap (MOI 400) were used with increasing amounts of rAAV-GFP input vector. (C and D) Please note that rep-rAdV corresponds to a different rAdV vector (rAd-HSV-TK-sRep1/530) than in figures 12 and 14 (rAd-M2-Tet-sRep78). (E) Anti-Cap western analysis and (F) silver staining of the medium-scale affinity purified rAAV-GFP preparations 1 and 2 (compare table 1), which were generated by rAd-HSV-TK-sRep-Stop531 and rAd-HSV-TK-Cap mediated amplification of starter rAAV-GFP. The numbers of purified rAAV-GFP genomic particles loaded are indicated.

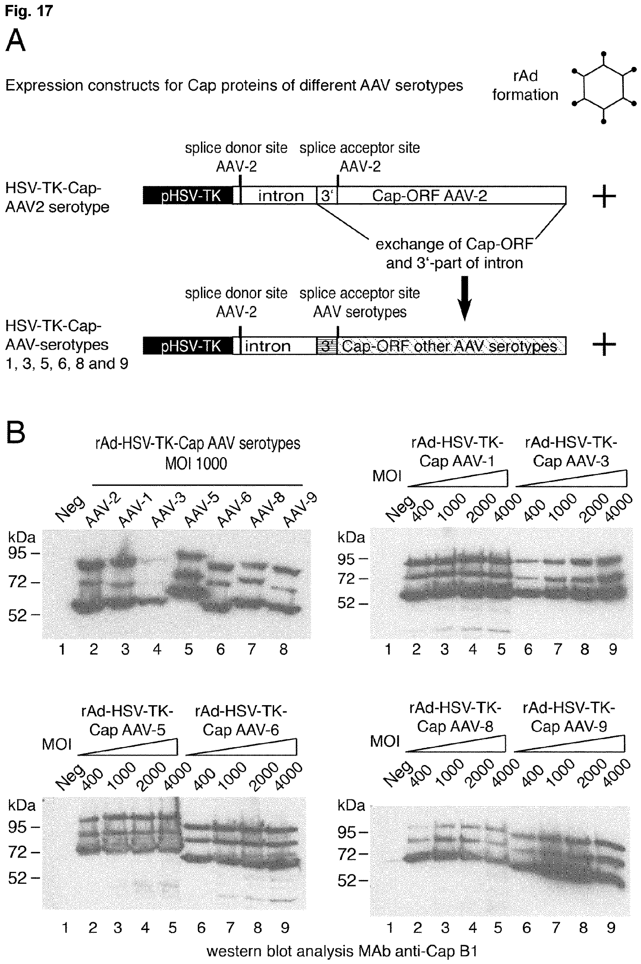

**Fig. 17** **Generation of adenoviral vectors (rAdVs) for the expression of cap proteins of different AAV serotypes.** (B) Cap expression after rAd-HSV-TK-Cap infection at different concentrations. (A) Schematic presentation of the adenoviral shuttle plasmids, which were generated by fusion of the 3-part of the intron of the respective AAV serotype and the downstream Cap ORF to the corresponding site in the AAV-2 intron of the pShuttle-HSV-TK-Cap AAV-2 construct. Different boxes denote the HSV-TK promoter, the 5'-part of the AAV-2 intron, the 3'-part of the AAV serotype introns and the respective Cap ORF. (B) Infection of HEK293 cells for 64 h with cap-rAdV vectors for AAV serotypes 1, 2, 3 , 5, 6, 8 and 9 at MOI 1000 (upper left) and 400 to 4000 as indicated (upper right and lower part of figure), followed by western analysis for Cap expression with anti-Cap MAb B1.

| **SEQ ID NO:** | **Description** |
|---|---|
| 1 | AAV-2 genome |
| 2 | RIS-Ad |
| 3 | Recoded RIS-Ad |
| 4 | sRep (recoded AAV-2 nucleotides 1782 to 1916) |
| 5 | Rep78 aa sequence |
| 6 | HSV-TK promoter |
| 7 | *rep*-rAdV nucleic acid sequence |
| 8 | Recoded p40 promoter 1 |
| 9 | Recoded p40 promoter 2 |
| 10 | *cap*-rAdV nucleic acid sequence |
| 11 | Example starter rAAV vector |
| 12 | Minimal RIS-Ad |
| 13 | Recoded minimal RIS-Ad |
| 14 | AAV-2 VP1 amino acid sequence |

### Examples

### Materials and methods

### Plasmids and cloning.

Plasmid pTR-UF5 (Zolotukhin et al., 1996) carrying a CMV enhancer/promoter driven GFP cassette flanked by the AAV-2 ITR's was kindly provided by Nick Muzyczka (University of Florida). The plasmids pShuttle-p5-Rep and pShuttle-p5-sRep containing the AAV-2 p5 promoter and the complete Rep78 coding region have been described (Weger et al., 2014). The term sRep denotes a Rep78 sequence with recoding of AAV-2 nt 1782 to 1916 according to Sitaraman et al. (Sitaraman et al., 2011). For generation of pShuttle-MMTV-Rep and pShuttle-MMTV-sRep, the sequences between the BgIII site preceding the p5 promoter and the unique Sall site within the Rep-ORF (AAV-2 nt 1428) in the corresponding pShuttle-p5-Rep constructs were replaced by a BamHI-partial/Sall restriction fragment from pDG (Grimm et al., 1998) containing the MMTV-LTR and the amino-terminal part of the Rep ORF. For cloning of pShuttle-M2-Tet-Rep constructs, a cassette containing the CMV promoter, the rtTA (M2) transactivator and the Tet promoter (an array of 7 Tet-Operator sequences followed by a CMV core promoter) was excised as Hindlll/Agel fragment from plasmid pD-Tet-M2-K1, and assembled with the corresponding Rep78 ORFs (either wildtype or sRep nucleotide sequence), generated as PCR fragments with flanking Agel/Xbal sites, in Hindlll/Xbal digested pShuttle vector by three-component ligation. Plasmids pShuttle-p40-Cap and pShuttle-s-p40-Cap have been described (Weger et al., 2014). For expression of the AAV-2 Cap proteins under the control of heterologous promoters CMV (*human cytomegalovirus early enhancer*/*promoter*), PGK (*murine phosphoglycerate kinase promoter*), HSV-TK (*herpes simplex virus thymidine kinase promoter*) and adenovirus subtype 5 MLP (*major late promoter*), the p40 promoter sequences in pShuttle-p40-Cap up to the HindIII site at AAV-2 nt 1882 were replaced by the corresponding heterologous promoter sequences amplified from suitable parental plasmids with primers generating Xbal and HindIII sites for cloning. The Ad-MLP sequence contains nt -506 to +192 with respect to the start site for the late Ad5 transcripts including the first leader sequence, while the sequence denoted as Ad-MLP-I additionally contains the complete first intron and the second leader sequence (nt -506 to 1134 with respect to the transcription start site). For generation of pShuttle-ITR-GFP containing an CBA (chicken β-actin CMV hybrid promoter) driven GFP gene flanked by the AAV-2 ITR's, the complete ITR-flanked transgene cassette was excised from pTR-UF26 (Mietzsch et al., 2014) with Pstl, first subcloned into pBluescript II SK (+) and then excised with EcoRV/Xbal for cloning into the pShuttle vector. An additional Pacl site present in a non-essential region of the transgene cassette was deleted by partial digestion, fill-in reaction and religation. pShuttle-HSV-TK-sRep78 was generated by exchanging the CMV-M2-Tet cassette in pShuttle-M2-Tet-sRep with the HSV-TK promoter amplified with primers generating HindIII and Agel sites for subcloning. For pShuttle-HSV-TK-sRep68 and pShuttle-HSV-TK-sRep1/530, the sRep78 cassette in pShuttle-HSV-TK-sRep78 was exchanged for the corresponding partially recoded Rep sequences through the Agel and Xbal sites flanking the Rep ORF. The sRep1/530 sequence was amplified from pShuttle-M2-Tet-sRep with primers generating an Agel site at the 5'-end and introducing the termination codon TAA downstream from Rep78 amino acid 530 and an Xbal site at the 3'-end, respectively. The sRep68 sequence was amplified from pShuttle-M2-Tet-sRep with primers generating an Agel site at the 5'-end and a primer encoding the first 3 Rep68-specific amino acids with a BssHII restriction in frame with Rep78 amino acid 529. The Agel/BssHII-cut amplification product was ligated to the Agel/Xbal cut vector using a BssHII/Xbal linker containing the remaining 4 Rep68-specific amino and a termination codon. All pAdEasy constructs were generated from the corresponding pShuttle plasmids through homologous recombination of Pmel linearized DNA in *E.coli* BJ5183-AD-1 cells (Agilent) containing the pAdEasy-1 plasmid (Genbank accession no. AY370909).

### Cell culture and transfection.

HEK293 (human embryonal kidney, ATCC CRL-1573) and HeLa (human cervix carcionoma) cells were propagated in Dulbecco's modified Eagle medium (DMEM) supplemented with 10% fetal calf serum (FCS) and 100 µg/ml of penicillin and streptomycin at 37°C with 5% CO₂. Transfections of HEK-293 cells were performed by the calcium phosphate precipitate technique as described (Winter et al., 2012). The numbers of cells seeded the day before transfection were 3 x 10⁵ cells for 6-well plates, 6 x 10⁵ cells for 25 cm² flasks or 4 x 10⁶ cells for 14.5 cm dishes, respectively.

### Generation and propagation of recombinant adenoviral vectors (rAdV).

Generation of rAdV was performed with the AdEasy system (He et al., 1998, Luo et al., 2007) largely as described. The corresponding pShuttle constructs were linearized with Pmel and electroporated into *E.coli* BJ5183-AD-1 cells for homologous recombination with the E1/E3 deleted Ad5 genome contained on the stably transformed pAdEasy plasmid. Recombinant pAdEasy plasmid DNA was amplified in *E.coli* XL-1-Blue cells, linearized with Pacl and transfected into HEK-293 cells in 25 cm² flasks. The primary rAdV preparation was harvested 12 to 14 days post transfection by four freeze-thaw cycles in liquid nitrogen/37°C water bath in 2 ml of medium. 1.5 ml of the supernatant was used for first round of amplification with 2 x 10⁶ cells seeded in 75 cm² flasks the day before infection. Further rounds of amplification and purification by CsCI gradient centrifugation were performed as described (Luo et al., 2007).

### Quantification of genomic rAdV particles.

Adenoviral genomic particles in rAdV freeze-thaw supernatants or CsCI purified rAdV were determined by Light-Cycler based Real Time PCR with the GoTaq qPCR Master Mix (Promega) using Primers E4-Q1 and E4-Q2 for amplification of a 202 bp region from the Ad5-E4 region as described recently (Weger et al., 2014).

### Infection of HEK-293 cells with recombinant adenoviruses (rAdV).

For western or southern blot analysis and production of rAAV, HEK-293 cells were infected in the appropriate cell culture vessels with the indicated amounts (MOI generally stated in genomic particles per cell) of rAdV freeze-thaw lysates or CsCI purified rAdV without prior removal of the cell culture media.

### Western analysis.

Immunoblot analysis of purified rAAV-2 preparations for capsid content or of whole cell extracts from transfected or rAdV infected HEK-293 cells for AAV-2 Cap or Rep expression was performed with anti-Cap monoclonal antibody B1 diluted 1:20 or anti-Rep monoclonal antibody 303.9 diluted 1:10 (both from Progen, Heidelberg, Germany), respectively, followed by incubation with horseradish peroxidase conjugated secondary antibodies and enhanced chemiluminescent detection (ECL) as described previously (Winter et al., 2012).

### Silver staining.

Capsid protein contents of purified rAAV-2 vector preparations were analyzed in silver stained gels with different amounts of vector, corresponding to 1.0 x 10⁸ to 1.0 x10¹⁰ genomic particles, directly lysed in protein sample buffer, incubated for 5 min at 95°C and separated on 8% polyacrylamide/SDS gels. The staining reaction was performed as described in (Mietzsch et al., 2014).

### Extraction of viral DNA and Southern hybridization.

Extraction of viral DNA by a modified Hirt procedure was performed essentially as described (Winter et al., 2012). Southern Blot analysis was performed with 6 µg of Hirt-DNA's digested with 40 units of *Dpn*I for 2 h. After electrophoresis on a 0.8 % agarose gel, DNA's were transferred by capillary blotting to a nylon membrane (Hybond-N, GE Healthcare) and hybridized with a Biotin-11-dUTP-labeled 1.9 kb EcoRI probe from pTR-UF5 comprising the CMV promoter, the GFP-ORF and the SV40 polyadenylation site. The bound biotinylated probe was detected with Streptavidin horseradish peroxidase conjugate (high sensitivity HRP Conjugate, Thermo Scientific) followed by enhanced chemiluminescent detection (ECL).

### Production and purification of rAAV in HEK-293 cells.

HEK-293 cells were seeded at 25 to 30% confluency. 24 hours later, cells were either (I) transfected with pTR-UF5 and additionally infected with the two rAdVfor Rep and Cap protein expression, (II) transduced with prepackaged starter rAAV-GFP and co-infected with the two rAdV for Rep and Cap protein expression or (IV) triple-infected with ITR-GFP-rAdV providing the ITR-flanked GFP vector DNA and the two rAdV for Rep and Cap protein expression. Cells co-transfected with the pDG helper and the pTR-UF5 GFP-expressing vector plasmid at a 1:1 molecular ratio were generally used as positive control (pc). For the approaches involving transfection, the cell medium was replaced by fresh medium 16 hours post transfection. Cells were generally harvested 64 hours post transfection/infection. For optimization of rAAV packaging in 6-well plates, the scraped cells were directly lysed in the medium by three freeze-thaw cycles and, after centrifugation, the supernatant was stored at -80°C fur further use. For medium scale rAAV production and purification, harvested cells were washed with PBS, resuspended in 1 ml of lysis buffer (10mM Tis-HCI pH 8.5, 150mM NaCl, 1 mM MgCl₂ and 1% Triton-X-100 v/v) per 14.5 cm dish and lysed by three freeze-thaw cycles. Crude lysates were treated with 250U benzonase (Merck) per ml lysate at 37°C for 1 hour to degrade unpackaged AAV DNA and centrifuged at 8,000g for 30 minutes to pellet cell debris. The supernatant was frozen at -80°C for at least 1 hour. After thawing and centrifugation at 8,000g for 15 minutes the supernatant was subjected to one-step AVB sepharose affinity chromatography using 1 ml prepacked HiTrap columns on a ÄKTA purifier system (GE Healthcare) as described (Mietzsch et al., 2014).

### Quantification of rAAV vector preparations.

Aliquots of affinity purified rAAV-GFP vector preparations were digested with proteinase K (Roth, Germany) at a final concentration of 400 µg/ml at 56°C for 2 hours in lysis buffer (1% [w/v] N-Lauroylsarcosine, 25 mM Tris pH 8.5, 10 mM EDTA pH 8.0) to release the vector genomes from the capsids. Following phenol-chloroform extraction and ethanol-precipitation, samples were dissolved in 20 µl of Tris-EDTA buffer pH 7.6 and prediluted 1:100 for the determination of genomic copy numbers by Light-Cycler based Real Time PCR with the GoTaq qPCR Master Mix (Promega). Primers specific for the bovine growth hormone derived polyA site of the vector backbones were used as described (Mietzsch et al., 2014).

*Determination of rAA V-GFP transduction efficiencies in HeLa cells by FACS analysis.* 3 x 10⁵ HeLa cells in 12-well plates were either transduced with small volumes of freeze-thaw supernatants from HEK-293 cells used for rAAV-GFP packaging (usually a volume corresponding to 1/15 of the original HEK-293 6-well was applied) or with affinity purified rAAV-GFP at the indicated MOIs (gp/cell), and superinfected with adenovirus type 2 at a MOI of 10 (infectious titer). Cells were harvested 44 hours post transduction, washed once with PBS and resuspended in 500 µl of 20% FCS in PBS. FACS analysis was performed with a FACS Calibur according to the manufacturer's protocol (Becton & Dickinson). The cut-off level for GFP-positive cells was determined with non-transduced, Ad2 infected HeLa cells as controls.

### Results

### Generation of stable recombinant adenoviruses (rAdV) expressing functional Rep proteins.

The RIS-Ad, a regulatory element in the AAV genome inhibiting adenoviral replication *in cis* (Fig. 9A), not only constitutes part of the Rep ORF, but it is also required for efficient Cap protein expression through control of p40 promoter activity and AAV-2 splicing (Weger et al., 2016). Our basic strategy for rAdV-mediated expression of AAV Rep and Cap proteins therefore relied on separation of these AAV genes onto two different vectors. For Rep protein expression, we impeded RIS-Ad function by recoding of Rep78 amino acids 488 to 531 (Fig. 9A, termed sRep). Since Rep78 has been shown to inhibit adenoviral replication *in trans* (Timpe et al., 2006, Weger et al., 2014), we used not only the physiological AAV-2 p5 promoter, but in parallel also the weaker MMTV-LTR (Fig. 9B, MMTV-Rep and MMTV-sRep) and an inducible system to drive its expression. The later consisted of a constitutive expression cassette for the tetracycline dependent transactivator (rtTA/M2) directly linked to the corresponding response element upstream of the Rep ORF (Fig. 9B, M2-Tet constructs). The small Rep proteins were generally expressed under control of the internal AAV-2 p19 promoter. When the pAdEasy plasmids were used for generation of rAdV, all constructs harboring the wildtype Rep sequence in repeated experiments did not give rise to rAdV (Fig. 9B). This finding confirmed the cis-inhibitory function of the RIS-Ad established in previous studies (Weger et al., 2016, Weger et al., 2014). The p5-sRep construct led to low titer rAdV supernatant preparations expressing small amount of the Rep proteins in the initial amplification rounds. However, reduction of viral titers and Rep protein expression observed in subsequent amplification rounds (data not shown) implied that the virus was not stable. In contrast, the initial rAdV preparations obtained from MMTV-sRep and M2-Tet-sRep constructs could be successfully passaged with titers in the range of 3 x 10¹¹ to 6 x 10¹¹ genomic particles (gp)/ml in freeze-thaw medium supernatants obtained after 5 to 6 amplification rounds. These titers were comparable to those of the rAdV-CMV-GFP control virus. By CsCI gradient density purification, the rAdV vectors could be concentrated to titers of 2 to 4 x 10¹² gp/ml. A side-by-side comparison of rAdV-mediated Rep expression levels was performed after these 5 to 6 amplification rounds, with protein extracts from cells transfected (at efficiencies of about 70%) with the well-established standard plasmid pDG for rAAV-2 vector production (Grimm et al., 1998) serving as positive control. Already at vector doses of 400 to 1000 gp/cell (Fig. 10A, upper panel, lanes 7 and 8), rAdV-M2-Tet-sRep showed similar Rep52 and slightly higher Rep78 levels than the positive control (lane 2), with no major further increase at vector doses of 2000 or 4000 gp/cell (lanes 9 and 10). At comparable multiplicities, rAdV-MMTV-sRep expressed lower Rep52 and especially Rep78 amounts with Rep78 levels in the range of the positive control only at the vector dose of 4000 gp/cell (Fig. 10A, upper panel, lanes 3 to 6). In contrast to earlier transfection experiments with the corresponding pShuttle and pAdEasy plasmids (data not shown), barely any doxycycline mediated induction of Rep78 expression was observed for rAdV-M2-Tet-sRep (Fig. 10A, lower panel). This may be due to early adenoviral functions stimulating the minimal CMV promoter, which constitutes part of the Tetracycline response element (TRE), since regulation was also lost after transfection of the pShuttle constructs in the presence of adenovirus infection (data not shown). The ability of rAdV-M2-Tet-sRep to promote the DNA replication of an AAV-2 ITR flanked GFP cassette was assayed by infection of pTR-UF5 transfected cells. After application of 2000 to 8000 gp/cell, abundance of replicative monomeric (RF-M) and dimeric (RF-D) intermediates was similarly high as that scored for the pDG/pTR-UF5 co-transfection positive control (Fig. 10B). Surprisingly, at high MOIs, addition of doxycycline rather reduced ITR-dependent replication (Fig. 10B, lanes 9 to 12). Lower levels of AAV-ITR dependent DNA replication were observed with rAdV-MMTV-sRep (data not shown). To provide the template for rAAV packaging not only by transfection, but also by an infection step, we generated an rAdV harboring an AAV-2-ITR flanked GFP transgene (rAdV-ITR-GFP). This ITR-flanked GFP transgene could also be replicated by rAdV-M2-Tet-sRep, with the typical monomeric and dimeric replicative forms detected both after transfection of the transfer vector (Fig. 10C, pShuttle-ITR-GFP, lanes 1 to 4) and after rAdV-ITR-GFP/rAd-M2-Tet-sRep co-infection (Fig. 10C, lanes 5 to 12).

### Recombinant Adenoviruses can mediate high-level expression of the AAV-2 Cap proteins under control of the HSV-TK promoter.

As the wildtype p40 promoter constitutes a major element of the RIS-Ad (Fig. 9A), it could not be used for generation of rAdVs expressing the AAV-2 Cap proteins (Weger et al., 2014). Different heterologous promoters (Fig. 11A) including the CMV, the PGK, the HSV-TK and additionally the adenovirus subtype 5 major late major late promoter (MLP) were therefore tested upstream of the AAV-2 intron for their ability to drive Cap protein expression. Two different versions of the MLP, differing only in the absence/presence of the first Ad5-derived intron (Fig. 11A, MLP-Cap and MLP-I-Cap), and additionally a mutated AAV-2 p40 promoter with the sRep sequence (Fig. 11A, s-p40-Cap) were included in the analysis. After transfection of the pAdEasy plasmids into HEK293 cells, in particular those with Cap expression controlled by the HSV-TK or the mutated AAV-2 p40 promoter eventually gave rise to rAdV genomic particles (Fig. 11A). Considering the hardly detectable VP expression levels of the pAdE-p40-Cap construct after transient transfection (data not shown) and the possible recombination of p40 promoter sequences with carboxy-terminal Rep sequences contained in the rep-rAdV, only the rAdV-HSV-TK-Cap was further pursued as means for providing the capsid proteins for rAAV packaging. Western blot analysis of protein extracts from HEK293 cells infected with medium supernatants from amplification rounds 3 to 8 showed high-level expression of the three capsid proteins VP1 to VP3 with a stoichiometry comparable to that of pDG transfection used as a positive control (Fig. 11B). Saturated capsid proteins levels were observed at rAd vector doses of 1000 to 4000 gp/cell (fig. 11C).

### Optimization of rAd-mediated packaging of rAAV vectors.

Expression of the AAV-2 Rep and Cap proteins by the newly generated rAdV was subsequently used to package an AAV-2 ITR flanked CMV-driven GFP transgene into recombinant AAV particles. In a broad optimization scheme using different MOIs of the rAdV, three different setups (Fig. 12, upper part) were compared to the well-established standard packaging system of co-transfecting a vector plasmid (pTR-UF5 with a CMV-GFP transgene cassette) with the helper plasmid pDG (Grimm et al., 1998), which was used as a positive control: (I) over-infection of pTR-UF5 transfected HEK293 cells with the two rAdV for Rep and Cap expression; (II) rep-rAdV/cap-rAdV mediated amplification of a purified starter rAAV-GFP preparation provided at low MOIs and finally (III) triple infection of an rAdV containing the ITR-flanked GFP transgene (ITR-GFP-rAdV) with the two rAdVs for Rep and Cap. The two latter systems (II and III) are independent of DNA transfection. In initial experiments (data not shown) the yields of transducing rAAV particles with rAdV-MMTV-sRep were generally clearly lower than those achieved with rAdV-M2-Tet-sRep. For further optimization, rAdV-M2-Tet-sRep was therefore exclusively used and for simplification purposes is denoted as rep-rAdV in the following and in fig. 12 (likewise rAdV-HSV-TK-Cap is also denoted only as cap-rAdV). No major differences were observed in the presence or absence of doxycycline and all experiments presented were performed in the absence of doxycycline. As read-out for rAAV vector production, transducing rAAV-GFP particles in freeze-thaw supernatants were determined by secondary transduction of HeLa cells. Representative experiments for each of the three setups with a positive control (standard cotransfection) included in each experiment are shown in the lower part of fig. 12. Generally, with optimized MOIs (given as GP/cell) of the viral components, similar amounts of transducing particles as in the positive control could be obtained for all three setups. For the rAdV-mediated starter rAAV-GFP amplification protocol, the number of transducing units could be amplified by a factor of 100 to 200 using freeze-thaw supernatants as a source of starter rAAV-GFP (fig. 12, II). Providing the GFP vector template as rAdV (setup III, rAd triple infection) also led to high yields of transducing rAAV-GFP particles with a clear decrease at higher MOIs of ITR-GFP-rAdV (shown for MOI 10000 in fig. 12, III). This is probably due to interference with the rAdVs for Rep and Cap expression as demonstrated by Western analysis (not shown). In summary, in crude freeze-thaw lysates all three systems employing the rAdV for expression of the AAV-2 Rep and Cap proteins led to similar or even better yields of transducing rAAV-GFP units as the standard co-transfection protocol.

### Yields and characterization of purified rAAV vector preparations generated with recombinant adenoviruses.

To more closely analyze the rAAV-2 vectors generated with the novel rAdV-dependent packaging systems, two independent medium scale rAAV-GFP vector preparations (8 x 10⁷ HEK293 cells at the time of transfection/infections) for each of the experimental setups described above (compare fig. 12, I to III) were performed under the optimized conditions, with two preparations of the standard co-transfection protocol run in parallel as positive controls. All rAAV-GFP preparations were subjected to one-step AVB affinity purification. In agreement with the data from the optimization experiments, the yields of the purified rAAV-GFP vector fractions were in a similar range of about 1 x 10⁴ GP/cell (burst size), regardless of the setup used for packaging (fig. 13A). AAV-2 wildtype genomes were below the detection limit of the PCR-based assay in all preparations. All purified vectors also showed similar transduction rates in HeLa cells over a wide range of MOIs, when normalized for genomic particles (fig. 13B). The only exceptions were the rAAV-GFP vectors produced by rAdV triple infections, which tended to lead to higher transduction rates. The latter finding may be due to a slightly different promoter (chicken β-actin CMV hybrid vs. CMV promoter) in these vectors due to the nature of the rAdV providing the vector template. With the rAdV-mediated starter rAAV amplification method (fig. 12, method II), an increase in the input number of genomic rAAV-GFP particles by a factor of about 100 to 200 and an increase in transducing particles of about 200 to 400 were achieved.

For the judgment of particle capsid content/composition and integrity, equal numbers of genomic particles from the respective preparation with the higher burst size for each of the four setups were subjected to SDS PAGE followed by silver staining. Background-free VP1-3 bands with apparently similar ratios of the individual capsid proteins were detected for all preparations (Fig. 13D) with the identities of the three major silver-stained bands confirmed in anti-Cap western blot analysis (Fig. 13C). However, the rAAV vector preparations generated by protocols I and III (pTR-UF5 transfection/rAdV overinfection and rAdV triple infection, respectively) showed an up to 10-fold higher capsid protein content than the one generated by the classical co-transfection protocol, pointing to a probable excess of empty particles.

### Stable amplification of the recombinant adenoviruses used for rAAV packaging.

An important requirement for the use of rAdV in reproducible packaging of rAAV vectors is their genomic integrity throughout stepwise amplification. To assess the genomic stability of the newly generated rAd vectors for AAV-2 Rep and Cap expression (rAdV-M2-Tet-sRep and rAdV-HSV-TK-Cap), these were passaged over 15 rounds with amplification factors of 50 to 150 achieved for each individual round of amplification, as determined by analysis of the amount of genomic particles. The genomic titers obtained in freeze-thaw lysates (with about 3.0 to 4.0 x 10⁶ cells lysed in 1 ml of medium) were in a largely constant range of 2 to 6 x 10¹¹ GP/ml for rAdV-M2-Tet-sRep (fig. 14A, left panel) after two and rAdV-HSV-TK-Cap (fig. 14A, right panel) after five amplification rounds, respectively. For both vectors, assessment of the integrity of the vectors by PCR-based analysis with primers spanning the complete transgene cassette revealed only one distinct major amplification product throughout the complete upper series (starting with round 4) of amplification rounds (fig. 14B, rep-rAdV in the left and cap-rAdV in the right panel). These bands corresponded in size to the respective full-length sequences, as also judged by comparison with the PCR products obtained from the respective pAdEasy plasmids used for generation of the rAd vectors (positive controls in fig. 14B). No lower-sized bands pointing to a possible deletion of parts of the transgene cassettes in subpopulations of the rAdV vectors were observed. These findings were confirmed by western blot analysis of Rep (Fig. 14C) and Cap (Fig. 14D) protein expression after infection of HEK293 cells with the rAdV supernatants from the sequential amplification rounds (again starting with round 4) at MOIs of 200. Furthermore, when comparing the ability of the *rep-*rAdV supernatants from amplification rounds 4 and 15 to support rAAV2-GFP vector packaging in small and medium scale setups, only minor differences within the range of variation of the experimental setup were found (data not shown). In summary, these findings show that the newly generated rAdV are highly stable with consistent Rep/Cap expression and support of rAAV packaging over continuous rounds of amplification in the standard cell line HEK293.

### Improved rAdV-mediated rAA V packaging using Carboxy-terminal deleted Rep proteins expressed under the control of the HSV-TK promoter.

As noted before, some of the rAAV2-GFP preparations packaged with the help of recombinant adenoviruses displayed a relatively high capsid content (see fig. 13C and D). We therefore reasoned that the abundance of the Cap proteins expressed from rAdV-HSV-TK-Cap probably did not represent a limiting factor for packaging. In contrast, DNA replication of the recombinant AAV vector genome after infection with rAdV-M2-Tet-sRep did not quite reach the level of the positive control, the standard co-transfection system (compare fig. 10B). In an effort to increase the expression rates of the large Rep proteins responsible for AAV DNA replication, we therefore replaced the CMV-M2-Tet control cassette with the constitutive HSV-TK promoter, which had already proven to be well suited for rAdV-mediated expression of the Cap proteins. At the same time, the AAV2 intron sequences, which comprise the carboxy-terminal part of Rep 78 and Rep52, were deleted to prevent possible inhibitory effects on adenoviral replication described for amino acid motifs encoded by the AAV2 intron (Di Pasquale and Chiorini, 2003). Two different constructs containing either the Rep68 ORF including the 7 Rep68/Rep40 specific carboxy-terminal amino acids or a Rep ORF terminating almost directly after the splice donor site were generated (fig. 15A, termed Rep68 and Rep1/530, respectively). As for the previous vectors, the Rep ORF was recoded starting with amino acid 488 (AAV-2 nucleotide 1782). The corresponding rAdVs could be successfully generated. Subsequent infection of HEK293 cells with supernatants obtained from the respective amplification round 5 led, in comparison to the earlier rAdV-M2-Tet-sRep78 vector, to clearly higher proportions of the corresponding large Rep proteins for both rAdV-HSV-TK-sRep68 and rAdV-HSV-TK-sRep1/530 (fig. 15B). In line with the higher expression levels of the respective large Rep protein, rAdV-HSV-TK-sRep68 and rAdV-HSV-TK-sRep1/530 also led to strong abundance of replicative intermediates after coinfection with a purified starter rAAV2-GFP vector as source of a recombinant AAV genome (fig. 15C). For MOIs of 200 and 1000, these levels of rAAV-GFP replicative intermediates were clearly higher than for the positive control, the pDG/pTR-UF5 cotransfection (fig. 15C).

For application of rAdV-HSV-TK-sRep68 and rAdV-HSV-TK-sRep1/530 in rAAV packaging, the approach of rAdV-mediated starter rAAV amplification (see fig. 12, II) was chosen. In initial small-scale experiments, the MOIs for the rAdVs for Cap and Rep expression as well as for the pre-purified starter rAAV-GFP vector were optimized with the number of transducing units obtained in raw freeze-thaw lysates from the packaging reactions as read-out. In these experiments we observed a sharp drop in the capability of rAdV-HSV-TK-sRep68 to promote rAAV packaging, when this rAdV was amplified beyond round 5, whereas the vector preparation from round 5 was comparable to that of AdV-HSV-TK-sRep1/530 (data not shown). This was accompanied by a loss in Rep expression levels for rAdV-HSV-TK-sRep68 starting with round 6 (data not shown). A PCR-analysis of the rAdV transgene cassette with primers located in the flanking adenoviral sequences revealed an additional band about 1.0 kb larger than the expected size of 2.5 kb, which was clearly seen in the material from amplification round 6 and became even more prominent in the material from round 7 (fig. 16A). This points to possible insertions within the transgene cassette. In contrast, for rAdV-HSV-TK-sRep1/530, only the band of the expected size was seen up to round 10, the highest amplification round investigated (fig. 16B). Therefore, all subsequent packaging experiments were therefore performed with rAdV-HSV-TK-sRep1/530. Providing both rAdV-HSV-TK-sRep1/530 and rAdV-HSV-TK-Cap at MOIs of 200 to 400 led to efficient starter rAAV-GFP amplification (fig. 16C, please note, that deviating from fig. 12 and 14, rep-rAdV now corresponds to rAdV-HSV-TK-sRep1/530). Further raising the MOI of only one of the two rAdVs was not beneficial, as transduction efficiencies even dropped. Applying both vectors at high MOIs (rAdV-HSV-TK-sRep1/530 at MOI 1000 and rAdV-HSV-TK-Cap at MOI 2000) at best marginally improved the yield of transducing units, while raising concerns about producing rAAVs with a high content of empty particles under these conditions. Applying the optimized MOIs (200 to 400 for the two rAdVs and 20 to 50 for the input rAAV-GFP), three independent medium-size rAAV-GFP amplifications were performed with subsequent affinity purification of rAAV particles. Burst sizes between 1.51 and 2.84 × 10⁴ GP/cell were achieved (table 1). These corresponded to amplification factors of the input numbers of genomic starter rAAV-GFP particles between 302 and 1419. The later was obtained for the preparation, where a starter rAAV-GFP MOI of only 20 was used. To assay particle capsid content and composition, two of the purified preparations were analyzed side by side to preparations obtained from the standard co-transfection protocol by Cap western analysis and silver staining. For one of the two preparations, capsid content was comparable to the respective positive control (fig. 16E, left panel). For the other one, capsid content was even lower (fig. 16E, right panel), pointing to a smaller proportion of empty capsids. Both rAAV-GFP preparations were free of contaminating proteins as judged by silver staining (fig. 16F). In summary, by co-infection at rather low MOIs with the improved rAdV-HSV-TK-sRep1/530 vector and the rAdV-HSV-TK-Cap vector we could amplify small amounts of pre-purified starter rAAV vector with high efficiency obtaining rAAV preparations with favorable properties. rAd-HSV-TK-sRep1/530 could be stably propagated without loss of genomic integrity (Fig. 16B) or Rep protein expression (data not shown) for at least 10 amplification rounds.

**Table 1: Characteristics of three independent starter rAAV-GFP serotype 2 amplifications performed with rAdV-HSV-TK-sRep1/530 and rAdV-HSV-TK-Cap (AAV-2)**

| **rAAV-GFP amplification** | **number of cells** | **Input MOI rAAV-GFP** | **titer (GP/ml) in 1 ml peak fraction** | **burst size (GP/cell) 1 ml peak fraction** | **fold amplification rAAV-GFP (GP)** |
|---|---|---|---|---|---|
| 1 | 8.0 x 10⁷ | 50 | 1.55 x 10¹² | 1.94 x 10⁴ | 388 |
| 2 | 1.6 x 10⁸ | 50 | 2.42 x 10¹² | 1.51 x 10⁴ | 302 |
| 3 | 1.6 x 10⁸ | 20 | 4.54 x 10¹² | 2.84 x 10⁴ | 1419 |

### Adaptation of rAd-mediated rAAV vector production to the capsid proteins of other AAV serotypes.

By co-expressing the AAV-2 Rep proteins with the capsid proteins of other serotypes, an AAV2-ITR based vector genome can also be packaged into the corresponding heterologous capsids. Such vectors are accordingly referred to as pseudotyped rAAV vectors (cf. also Fig. 4, "rAAV-2/X", where "X" stands for the serotype of the capsid proteins used). In order to apply the newly developed rAdV-based rAAV-2 packaging methods to pseudotyped rAAV-2/X vectors, rAdV vectors for the expression of the capsid proteins of AAV serotypes 1, 3, 5, 6, 8 and 9 were generated. The inhibition of adenoviral replication by the regulatory sequences for capsid expression localized in the 3' part of the AAV rep gene does not represent a serotype 2-specific phenomenon, but also applies to the corresponding genome regions of other AAV serotypes (Weger et al., 2016). Accordingly, the capsid proteins of alternative AAV serotypes were also placed under the control of the HSV-TK promoter.

Downstream of the HSV-TK promoter, the 5'-part of the AAV-2 intron was fused with the 3'-part of the intron of the alternative serotypes followed by the corresponding downstream located capsid reading frame, with the fusion site located directly upstream of the pyrimidine-rich region of the respective splice acceptor sites (Fig. 17A). For all AAV serotypes mentioned, rAdV with the respective HSV-TK driven capsid reading frame could be stably propagated over at least six amplification rounds. Already at an MOI of 400 GP/cell, these rAdV led to a strong expression of the respective capsid proteins (Fig. 17B). Thereby, the three capsid proteins VP1, VP2 and VP3 were expressed in a correct size and stoichiometry for all cloned serotypes. In agreement with the literature, the capsid proteins of AAV serotypes 5 and 8 showed a significantly different migration behavior compared to the other serotypes. With the exception of the rAdV for the capsid proteins of AAV serotype 3, a clear saturation of capsid expression was already observed from an MOI of 1000 GP/cell.

The expression rate was significantly higher than that after transfection (transfection efficiency was in the range of 50 to 70% positive cells) of the corresponding standard helper plasmids (Grimm et al., 2003), which express the capsid proteins under the control of the AAV-2 p40 promoter (data not shown).

By co-infection with rAdV-Tet-M2-sRep78 or rAdV-HSV-TK-sRep1/530, these rAdV for the expression of the capsid proteins of AAV serotypes 1, 3, 5, 6, 8 and 9 were successfully employed for packaging of the corresponding pseudotyped rAAV vectors by means of the set-ups I (providing the AAV-2 ITR flanked CMV-GFP transgene by plasmid transfection) and II (amplification of a starter rAAV-2-CMV-GFP vector) as outlined in fig. 12 for rAAV serotype 2 packaging.

After optimization, similar yields of transducing rAAV vector particles could be achieved with the rAdV-based methods as with the respective standard co-transfection approach for all serotypes examined (data not shown). For rAAV2-5 vectors, the yields obtained with both the rAdV-based methods and the standard plasmid co-transfection approach were quite low. This result can be explained by the described lack of compatibility of the AAV2 Rep proteins and the AAV-2 AAV-2 ITRs with the AAV5 capsid structure (Chiorini et al., 1999a, Chiorini et al., 1999b). Also in initial medium-scale assays with chromatographic purification of the rAAV vector particles as described above for rAAV-2 vectors, the yields in genomic particles were comparable to or higher than those of the respective positive controls.

These findings implicate that the rAdV-based rAAV packaging set-ups can be conveniently applied to the common AAV serotypes and capsid variants.

### Discussion

The successful application of AAV based vectors for the treatment of a variety of human genetic diseases has led to an increasing demand for rAAV large-scale production systems. Pre-clinical studies for toxicology, safety, dose, and bio-distribution assessments alone may often require up to 1E15 to 1E16 vector genomes, especially for systemic application in large animal models (Clement and Grieger, 2016). Whereas many vectors used in clinical trials are still produced by suspension cell adapted classical plasmid co-transfection protocols, exclusively infection-based rAAV packaging systems may be advantageous in terms of easy handling and the lack of requirement for large amounts of highly purified plasmid DNA (Clement and Grieger, 2016, Grieger et al., 2016). With mammalian cells, such systems have been implemented successfully with recombinant herpes simplex viruses (rHSV) (Conway et al., 1999) and adapted to large-scale production for use in clinical studies (Clement et al., 2009). Although adenovirus (Ad) presents the prototypical (Atchison et al., 1965) and best-characterized AAV helper virus, rAAV packaging systems based exclusively on first generation recombinant adenoviruses (rAdV) have so far struggled with limited replication and stability of the rAdVs expressing the AAV Rep proteins (Zhang et al., 2001).

One of the keys to our novel rAdV-based system was the functional inactivation of an AAV-2 sequence in the 3'-part of the rep gene, the RIS-Ad, which inhibits adenoviral replication in *cis* (Sitaraman et al., 2011, Weger et al., 2016). Since the RIS-Ad possesses a dual function in AAV-2 replication as part of the Rep coding region on one side and as control region for p40 promoter driven cap transcription on the other side, we allocated the Rep and Cap ORFs to separate rAdVs. This approach also offers a high flexibility with regard to adjusting the relative Rep and Cap expression levels and facilitates the adaption of the system to non AAV-2 serotype vectors or capsid variants. Recoding of the Rep ORF in a closely confined region (denoted as sRep) within the RIS-Ad allowed stable rAdV-mediated expression of the Rep proteins. In addition to the RIS-Ad acting as a *cis* inhibitory sequence, the large Rep proteins Rep78/Rep68 may limit adenoviral replication *in trans* (Timpe et al., 2006, Weger et al., 2014). Therefore, the promoters for Rep78 expression were initially chosen with regard to low (MMTV-LTR) or tightly regulated (M2-Tet) expression, as reduced Rep78/Rep68 expression under control of the MMTV-LTR has also been shown to be advantageous for the production of recombinant AAV particles with the classical co-transfection system (Grimm et al., 1998). The validity of these considerations was confirmed by our failure to stably propagate the rAdV expressing the recoded Rep78 under p5 promoter control, although this limited stability may also involve interactions of Rep78 with multifunctional Rep binding elements in the p5 region (Murphy et al., 2007). The strong induction of the CMV-M2-Tet mediated sRep78 expression by doxycycline seen with the pShuttle transfer plasmids was lost with the corresponding rAdV. The reasons are not quite clear, as analogous systems have been shown to retain induction of a recoded Rep78 protein (Sitaraman et al., 2011). Of note, in the later system, the expression of the M2 transactivator was driven by the HSV-TK promoter and not by the CMV promoter as in our constructs. Despite the low Rep78 induction, the ratio of Rep78 to Rep52 in rAdV-M2-Tet-sRep was similar to that seen for the pDG plasmid optimized for the rAAV-2 packaging cotransfection protocol (Grimm et al., 1998), which was used as positive control throughout our studies. However, DNA replication of a recombinant AAV genome by rAdV-M2-Tet-sRep seemed to be slightly reduced as compared to this positive control. Compared to the pDG transfection, the MMTV promoter led to clearly lower Rep78/Rep52 ratios after infection with the corresponding rAdV. Consequently, rAdV-MMTV-sRep was not as effective in promoting rAAV-DNA replication and packaging as rAdV-M2-Tet-sRep, and was therefore not further pursued. In the course of our studies, further improvements of rAdV-mediated rAAV packaging were achieved by using the HSV-TK promoter for expression of large Rep proteins devoid of the sequences encoded by the AAV-2 intron. These were fully functional in supporting rAAV-DNA replication and could be expressed at high levels in the context of rAdVs. AAV-2 intron encoded protein domains have been shown to suppress adenoviral replication by inhibiting PKA activity through direct protein-protein interactions (Di Pasquale and Chiorini, 2003). In line with these findings, we were unable to stably propagate a rAdV with a recoded Rep78 protein expressed under control of the HSV-TK promoter (data not shown). The use of the HSV-TK promoter for rAdV mediated expression of AAV-2 functions was another key component of our novel system as, of a series of cellular and viral promoters tested, it was also the only one found to lead to stable and high-level expression of the AAV-2 capsid proteins. It is hypothesized that this heterologous viral promoter is especially suitable for rAd-mediated expression of AAV-2 function, because of a high refractiveness to inhibition by adenoviral or AAV functions.

The large excess of empty over genome containing rAAV particles, as judged by capsid content, seemed to be one of the major drawbacks of our initial rAdV-M2-Tet-sRep based rAAV packaging systems. Empty particles have the potential to increase innate and adaptive immune responses after vector application (Mingozzi and High, 2013). Downstream purification of clinical grade vector preparations usually involves removal of such empty particles, e.g. by ion-exchange chromatography (Qu et al., 2007). Importantly, the advanced system involving rAdV-HSV-TK-sRep1/530 led to a higher replication of the rAAV genome and resulted in similar or even lower proportions of empty particles than the standard co-transfection protocol. Of note, a certain amount of empty particles may even be beneficial for rAAV transduction *in vivo.* An up to 100-fold excess of empty capsids serving as decoy was used to overcome preexisting B-cell mediated humoral immunity, a major hurdle to systemic rAAV delivery, without loss of transduction efficiency in factor IX gene therapy (Mingozzi et al., 2013). In this regard we could demonstrate that high-titer viral like particles (VLP) preparations may be efficiently produced by infection with rAdV-HSV-TK-Cap alone (not shown). In general, the expression of Rep and Cap from two separate rAdVs offers the possibility to adjust the capsid levels and thereby most probably also the proportion of empty particles over a wide range.

To routinely use rAdVs in large-scale rAAV vectors packaging, these must display a high genomic stability throughout multiple amplification steps. Of note, for a rAd vector containing the combined wildtype Rep and Cap expression cassette, a partial loss of this cassette within only a few amplification rounds was reported (Zhang et al., 2001). In contrast, our rAdV were completely stable for genomic integrity and transgene expression over at least 15 amplification rounds for rAdV-M2-Tet-sRep and rAdV-HSV-TK-Cap and at least 10 amplification rounds in case of rAdV-HSV-TK-sRep1/530. The later vector for improved expression of a large functional Rep protein has not been tested beyond this tenth amplification round. For the rAdV-HSV-TK-sRep68 vector containing 7 additional carboxy-terminal amino acids as compared to rAdV-HSV-TK-sRep1/530, we observed a reduction of Rep expression and support of rAAV packaging starting with amplification round 6. This was also experienced after re-amplification of material from earlier rounds (date not shown). Quite surprisingly, no deletion of parts of the transgene cassette occurred during the amplification, but a larger band appeared in the PCR-analysis, pointing to possible duplications of transgene sequences. The phosphorylation of Rep68 at one of the unique 7 carboxy-terminal amino acids, ⁵³⁵S, mediates its interaction with members of the 14-3-3 protein family and a Rep68-S535A point mutant is more efficient in promoting AAV DNA replication than the wildtype protein (Han et al., 2004). We have not elucidated yet, whether the putative duplication(s) within the rAd-transgene cassette may be due to such an interaction. However, we have firmly established that the partly recoded sRep1/530 protein missing the 7 Rep68 unique amino acids was fully functional for rAAV DNA replication and could be stably propagated within an rAdV.

With the initial rAdV-M2-Tet-sRep, the burst sizes for the different rAdV-based rAAV production schemes after purification, taking into consideration the peak fraction only, were in a range of 7 × 10³ to 1.6 × 10⁴ vg/c (vector genomes/cell) and thus very similar to the standard plasmid co-transfection system at high transfection efficiencies. This was regardless of whether the rAAV template was provided by transfection of a plasmid, by infection with small amounts of a starter rAAV vector or by a rAd/rAAV hybrid vector. The improved system using the carboxy-terminal deleted sRep1/530 protein led to at least two times higher yields using amplification of the starter rAAV vector, resulting in burst sizes of almost 3 × 10⁴ vg/c after affinity purification. Optimized rHSV-based rAAV production systems presently exhibit maximal burst sizes from 8 × 10⁴ to 1 × 10⁵ vg/c in crude cell lysates or supernatants (Chulay et al., 2011, Thomas et al., 2009), resulting in final yields after purification of large-scale preparations of about 2.0 × 10⁴ vg/c (Chulay et al., 2011, Clement and Grieger, 2016). The original Sf9 insect cell-based rAAV production system employing several recombinant baculoviruses (rBac) for Rep/Cap expression and delivery of the transgene (Urabe et al., 2002) routinely results in rAAV burst sizes of 2 × 10⁴ vg/c in bioreactor scale productions (Cecchini et al., 2011). With *Sf9* cell lines containing the AAV *rep* and cap genes stably integrated into the host genome, rAAV-2 yields of up to 1.4 × 10⁵ vg/c after iodixanol gradient centrifugation (Aslanidi et al., 2009) and 9 × 10⁴ vg/c in crude cell lysates (Mietzsch et al., 2015) have been described.

Despite not much obvious differences in vector yields in comparison to the well established rHSV- and Baculovirus based systems for large-scale rAAV production, we nevertheless reckon that our novel rAdV-based protocol may present an advantageous alternative. As one of the main advantages, the corresponding rAdV can be easily amplified to high titers without loss of Rep or Cap expression by serial passages in the standard HEK293 cell line. In contrast, the HSV based system relies on ICP27 deleted rHSV vectors, which have to be complemented by a corresponding cell line for propagation leading to rather low yields of the required rHSV components (Clement et al., 2009). Furthermore, rHSV generally are not as stable and easy to handle as first generation adenoviral vectors. The recombinant baculoviruses expressing the Rep and Cap proteins, which were used in the first generation rAAV packaging system in insect cells, are genetically unstable (Kohlbrenner et al., 2005, Smith et al., 2009). Whereas this problem was addressed by the aforementioned *Sf9 rep* and cap packaging cell lines, a major challenge remaining with the insect cell based systems are the significantly higher particle-to-infectivity ratios reported (>1000) as compared with rHSV or plasmid co-transfection generated rAAV. This may be due to the difficulty to express the three capsid proteins at correct ratios (Kohlbrenner et al., 2005, Urabe et al., 2002), with limiting VP1 amounts expressed for some AAV serotypes (Mietzsch et al., 2015). Differential post-translational modifications of the AAV capsid protein in mammalian and insect cells may also contribute to the lower transduction efficiencies of vectors produced in insect cells (Rumachik et al., 2020).

We have studied two different infection-based approaches to provide the rAAV template for the rAdV-mediated packaging. One relies on a third rAdV providing the AAV-ITR flanked transgene cassette, whereas in the other approach small amounts of a starter rAAV vector are amplified. For several reasons we would favor the later system for a simplified infection-based large-scale rAAV production system. The prepackaged rAAV starter vector can be produced using the standard cotransfection process. This means that transgene cassettes with novel therapeutic genes and/or control elements could be put into the large-scale production process directly after cloning into an AAV-ITR-based vector plasmid and an initial characterization. The time-consuming generation of new transgene-specific helper viruses or packaging lines, as required for other infection-based rAAV packaging systems, can be omitted. In addition to the need to generate a novel rAdV for each transgene to be packaged, the rAdV triple infection system also posed some issues with regard to reproducible handling. Competition effects between the different rAdVs are more pronounced with three individual rAdVs than with only two, rendering optimization quite difficult. This can be seen, for example in fig. 12, panel III, where higher amounts of the ITR-GFP-rAdV led to strongly reduced yields of rAAV vector, most probably because of reduced Rep and Cap protein levels from the other two rAdV (not shown). In contrast, the amount of input rAAV vector in the rAAV amplification scheme could be varied over a wide range without loss of packaging efficiency (fig. 16D). Whereas rAd/rAAV hybrid vectors have already been used in large scale rAAV preparations (Zhang et al., 2009), we also encountered some drop in rAAV packaging efficiency with increasing amplification rounds (data not shown).

As demonstrated by our study, the separate expression of the AAV2 Rep and Cap proteins from two stable first-generation recombinant adenoviruses holds great promise for large scale rAAV production. The adaption of this packaging system to HEK293 suspension cells and/or the extension fo further rAAV serotypes and capsid variants is possible.

### References

ASLANIDI, G., LAMB, K. & ZOLOTUKHIN, S. 2009. An inducible system for highly efficient production of recombinant adeno-associated virus (rAAV) vectors in insect Sf9 cells. Proc Natl Acad Sci U S A, 106, 5059-64.
ATCHISON, R. W., CASTO, B. C. & HAMMON, W. M. 1965. Adenovirus-Associated Defective Virus Particles. Science, 149, 754-6.
Becerra, S. P., F. Koczot, P. Fabisch, and J. A. Rose. 1988. Synthesis of adenoassociated virus structural proteins requires both alternative mRNA splicing and alternative initiations from a single transcript. J Virol 62:2745-54.
Carlson, C. A., D. M. Shayakhmetov, and A. Lieber. 2002. An adenoviral expression system for AAV rep78 using homologous recombination. Mol Ther 6:91-8.
CECCHINI, S., VIRAG, T. & KOTIN, R. M. 2011. Reproducible high yields of recombinant adeno-associated virus produced using invertebrate cells in 0.02- to 200-liter cultures. Hum Gene Ther, 22, 1021-30.
CHIORINI, J. A., AFIONE, S. & KOTIN, R. M. 1999a. Adeno-associated virus (AAV) type 5 Rep protein cleaves a unique terminal resolution site compared with other AAV serotypes. J Virol, 73, 4293-8.
CHIORINI, J. A., KIM, F., YANG, L. & KOTIN, R. M. 1999b. Cloning and characterization of adeno-associated virus type 5. J Virol, 73, 1309-19.
CHULAY, J. D., YE, G. J., THOMAS, D. L., KNOP, D. R., BENSON, J. M., HUTT, J. A., WANG, G., HUMPHRIES, M. & FLOTTE, T. R. 2011. Preclinical evaluation of a recombinant adeno-associated virus vector expressing human alpha-1 antitrypsin made using a recombinant herpes simplex virus production method. Hum Gene Ther, 22, 155-65.
CLEMENT, N. & GRIEGER, J. C. 2016. Manufacturing of recombinant adeno-associated viral vectors for clinical trials. Mol Ther Methods Clin Dev, 3, 16002.
CLEMENT, N., KNOP, D. R. & BYRNE, B. J. 2009. Large-scale adeno-associated viral vector production using a herpesvirus-based system enables manufacturing for clinical studies. Hum Gene Ther, 20, 796-806.
CONWAY, J. E., RHYS, C. M., ZOLOTUKHIN, I., ZOLOTUKHIN, S., MUZYCZKA, N., HAYWARD, G. S. & BYRNE, B. J. 1999. High-titer recombinant adeno-associated virus production utilizing a recombinant herpes simplex virus type I vector expressing AAV-2 Rep and Cap. Gene Ther, 6, 986-93.
DI PASQUALE, G. & CHIORINI, J. A. 2003. PKA/PrKX activity is a modulator of AAV/adenovirus interaction. EMBO J, 22, 1716-24.
Earley, L. F., J. M. Powers, K. Adachi, J. T. Baumgart, N. L. Meyer, Q. Xie, M. S. Chapman, and H. Nakai. 2017. Adeno-associated Virus (AAV) Assembly-Activating Protein Is Not an Essential Requirement for Capsid Assembly of AAV Serotypes 4, 5, and 11. J Virol 91.
Gabriel N, Hareendran S, Sen D, Gadkari RA, Sudha G, Selot R, Hussain M, Dhaksnamoorthy R, Samuel R, Srinivasan N, Srivastava A, Jayandharan GR. Bioengineering of AAV2 capsid at specific serine, threonine, or lysine residues improves its transduction efficiency in vitro and in vivo. Hum Gene Ther Methods. 2013 Apr;24(2):80-93. doi: 10.1089/hgtb.2012.194. Epub 2013 Mar 15. PMID: 23379478; PMCID: PMC3732126.
A Girod, M Ried, C Wobus, H Lahm, K Leike, J Kleinschmidt, G Deléage, M Hallek. 1999. Genetic capsid modifications allow efficient re-targeting of adeno-associated virus type 2. Nat Med. 5(9) 1052-6.
GRIEGER, J. C., SOLTYS, S. M. & SAMULSKI, R. J. 2016. Production of Recombinant Adeno-associated Virus Vectors Using Suspension HEK293 Cells and Continuous Harvest of Vector From the Culture Media for GMP FIX and FLT1 Clinical Vector. Molecular therapy: the journal of the American Society of Gene Therapy, 24, 287-297.
GRIMM, D., KAY, M. A. & KLEINSCHMIDT, J. A. 2003. Helper virus-free, optically controllable, and two-plasmid-based production of adeno-associated virus vectors of serotypes 1 to 6. Mol Ther, 7, 839-50.
GRIMM, D., KERN, A., RITTNER, K. & KLEINSCHMIDT, J. A. 1998. Novel tools for production and purification of recombinant adenoassociated virus vectors. Hum Gene Ther, 9, 2745-60.
HAN, S. I., KAWANO, M. A., ISHIZU, K., WATANABE, H., HASEGAWA, M., KANESASHI, S. N., KIM, Y. S., NAKANISHI, A., KATAOKA, K. & HANDA, H. 2004. Rep68 protein of adeno-associated virus type 2 interacts with 14-3-3 proteins depending on phosphorylation at serine 535. Virology, 320, 144-55.
HE, T. C., ZHOU, S., DA COSTA, L. T., YU, J., KINZLER, K. W. & VOGELSTEIN, B. 1998. A simplified system for generating recombinant adenoviruses. Proc Natl Acad Sci U S A, 95, 2509-14. https://en.wikipedia.org/wiki/Multiplicity_of_infection
KING ET AL. 2001, DNA helicase-mediated packaging of adeno-associated virus type 2 genomes into preformed capsids. Embo J 20, 3282-91.
KOHLBRENNER, E., ASLANIDI, G., NASH, K., SHKLYAEV, S., CAMPBELL-THOMPSON, M., BYRNE, B. J., SNYDER, R. O., MUZYCZKA, N., WARRINGTON, K. H., JR. & ZOLOTUKHIN, S. 2005. Successful production of pseudotyped rAAV vectors using a modified baculovirus expression system. Mol Ther, 12, 1217-25.
LUO, J., DENG, Z. L., LUO, X., TANG, N., SONG, W. X., CHEN, J., SHARFF, K. A., LUU, H. H., HAYDON, R. C., KINZLER, K. W., VOGELSTEIN, B. & HE, T. C. 2007. A protocol for rapid generation of recombinant adenoviruses using the AdEasy system. Nat Protoc, 2, 1236-47.
MCCARTY ET AL Identification of linear DNA sequences that specifically bind the adeno-associated virus Rep protein., 1994 J. Virol 68, 4988-4997.
MENDEL ET AL. 2017. Single-Dose Gene-Replacement Therapy for Spinal Muscular Atrophy. N Engl J Med. 377, 1713-1722.
MIETZSCH, M., CASTELEYN, V., WEGER, S., ZOLOTUKHIN, S. & HEILBRONN, R. 2015. OneBac 2.0: Sf9 Cell Lines for Production of AAV5 Vectors with Enhanced Infectivity and Minimal Encapsidation of Foreign DNA. Hum Gene Ther, 26, 688-97.
MIETZSCH, M., GRASSE, S., ZURAWSKI, C., WEGER, S., BENNETT, A., AGBANDJE-MCKENNA, M., MUZYCZKA, N., ZOLOTUKHIN, S. & HEILBRONN, R. 2014. OneBac: platform for scalable and high-titer production of adeno-associated virus serotype 1-12 vectors for gene therapy. Hum Gene Ther, 25, 212-22.
MINGOZZI, F., ANGUELA, X. M., PAVANI, G., CHEN, Y., DAVIDSON, R. J., HUI, D. J., YAZICIOGLU, M., ELKOUBY, L., HINDERER, C. J., FAELLA, A., HOWARD, C., TAI, A., PODSAKOFF, G. M., ZHOU, S., BASNER-TSCHAKARJAN, E., WRIGHT, J. F. & HIGH, K. A. 2013. Overcoming preexisting humoral immunity to AAV using capsid decoys. Sci Transl Med, 5, 194ra92.
MINGOZZI, F. & HIGH, K. A. 2013. Immune responses to AAV vectors: overcoming barriers to successful gene therapy. Blood, 122, 23-36.
Muralidhar, S., S. P. Becerra, and J. A. Rose. 1994. Site-directed mutagenesis of adeno-associated virus type 2 structural protein initiation codons: effects on regulation of synthesis and biological activity. J Virol 68:170-6.
MURPHY, M., GOMOS-KLEIN, J., STANKIC, M. & FALCK-PEDERSEN, E. 2007. Adeno-associated virus type 2 p5 promoter: a rep-regulated DNA switch element functioning in transcription, replication, and site-specific integration. J Virol, 81, 3721-30.
Ojala DS, Sun S, Santiago-Ortiz JL, Shapiro MG, Romero PA, Schaffer DV. In Vivo Selection of a Computationally Designed SCHEMA AAV Library Yields a Novel Variant for Infection of Adult Neural Stem Cells in the SVZ. Mol Ther. 2018 Jan 3;26(1):304-319. doi: 10.1016/j.ymthe.2017.09.006. Epub 2017 Sep 8. PMID: 28988711; PMCID: PMC5762983.
PEREIRA AND MUZYCZKA, 1997, The cellular transcription factor SP1 and an unknown cellular protein are required to mediate Rep protein activation of the adeno-associated virus p19 promoter. J Virol 71, 1747-1756.
QU, G., BAHR-DAVIDSON, J., PRADO, J., TAI, A., CATANIAG, F., MCDONNELL, J., ZHOU, J., HAUCK, B., LUNA, J., SOMMER, J. M., SMITH, P., ZHOU, S., COLOSI, P., HIGH, K. A., PIERCE, G. F. & WRIGHT, J. F. 2007. Separation of adeno-associated virus type 2 empty particles from genome containing vectors by anion-exchange column chromatography. J Virol Methods, 140, 183-92.
Recchia, A., R. J. Parks, S. Lamartina, C. Toniatti, L. Pieroni, F. Palombo, G. Ciliberto, F. L. Graham, R. Cortese, N. La Monica, and S. Colloca. 1999. Sitespecific integration mediated by a hybrid adenovirus/adeno-associated virus vector. Proc Natl Acad Sci U S A 96:2615-20.
Recchia, A., L. Perani, D. Sartori, C. Olgiati, and F. Mavilio. 2004. Site-specific integration of functional transgenes into the human genome by adeno/AAV hybrid vectors. Mol Ther 10:660-70.
REDEMANN ET AL., 1989, Adeno-associated virus rep protein synthesis during productive infection. J Virol 63 873-882.
RUMACHIK, N. G., MALAKER, S. A., POWELEIT, N., MAYNARD, L. H., ADAMS, C. M., LEIB, R. D., CIROLIA, G., THOMAS, D., STAMNES, S., HOLT, K., SINN, P., MAY, A. P. & PAULK, N. K. 2020. Methods Matter: Standard Production Platforms for Recombinant AAV Produce Chemically and Functionally Distinct Vectors. Molecular therapy Methods & clinical development, 18, 98-118.
SCHMIDT, M., CHIORINI, J. A., AFIONE, S. & KOTIN, R. 2002. Adeno-associated virus type 2 Rep78 inhibition of PKA and PRKX: fine mapping and analysis of mechanism. J Virol, 76, 1033-42.
Sen, D., Balakrishnan, B., Gabriel, N. et al. Improved adeno-associated virus (AAV) serotype 1 and 5 vectors for gene therapy. Sci Rep 3, 1832 (2013). https://doi.org/10.1038/srep01832.
SITARAMAN, V., HEARING, P., WARD, C. B., GNATENKO, D. V., WIMMER, E., MUELLER, S., SKIENA, S. & BAHOU, W. F. 2011. Computationally designed adeno-associated virus (AAV) Rep 78 is efficiently maintained within an adenovirus vector. Proc Natl Acad Sci U S A, 108, 14294-9.
SMITH, R. H., LEVY, J. R. & KOTIN, R. M. 2009. A simplified baculovirus-AAV expression vector system coupled with one-step affinity purification yields high-titer rAAV stocks from insect cells. Mol Ther, 17, 1888-96.
Sonntag, F., K. Kother, K. Schmidt, M. Weghofer, C. Raupp, K. Nieto, A. Kuck, B.Gerlach, B. Bottcher, O. J. Muller, K. Lux, M. Horer, and J. A. Kleinschmidt. 2011. The assembly-activating protein promotes capsid assembly of different adeno-associated virus serotypes. J Virol 85:12686-97.
Sonntag, F., K. Schmidt, and J. A. Kleinschmidt. 2010. A viral assembly factor promotes AAV2 capsid formation in the nucleolus. Proc Natl Acad Sci U S A 107:10220-5.
Sullivan JA, Stanek LM, Lukason MJ, Bu J, Osmond SR, Barry EA, O'Riordan CR, Shihabuddin LS, Cheng SH, Scaria A. Rationally designed AAV2 and AAVrh8R capsids provide improved transduction in the retina and brain. Gene Ther. 2018 Jun;25(3):205-219. doi: 10.1038/s41434-018-0017-8. Epub 2018 May 22. PMID: 29785047.
THOMAS, D. L., WANG, L., NIAMKE, J., LIU, J., KANG, W., SCOTTI, M. M., YE, G. J., VERES, G. & KNOP, D. R. 2009. Scalable recombinant adeno-associated virus production using recombinant herpes simplex virus type 1 coinfection of suspension-adapted mammalian cells. Hum Gene Ther, 20, 861-70.
TIMPE, J. M., VERRILL, K. C. & TREMPE, J. P. 2006. Effects of adeno-associated virus on adenovirus replication and gene expression during coinfection. J Virol, 80, 7807-15.
URABE, M., DING, C. & KOTIN, R. M. 2002. Insect cells as a factory to produce adeno-associated virus type 2 vectors. Hum Gene Ther, 13, 1935-43.
WARD ET AL., 1994, Adeno-associated virus DNA replication in vitro: activation by a maltose binding protein/Rep 68 fusion protein. J Virol 68, 6029-37.
WEGER, S., HAMMER, E., GONSIOR, M., STUTIKA, C. & HEILBRONN, R. 2016. A Regulatory Element Near the 3' End of the Adeno-Associated Virus rep Gene Inhibits Adenovirus Replication in cis by Means of p40 Promoter-Associated Short Transcripts. J Virol, 90, 3981-93.
WEGER, S., HAMMER, E. & HEILBRONN, R. 2014. Differential contribution of adeno-associated virus type 2 Rep protein expression and nucleic acid elements to inhibition of adenoviral replication in cis and in trans. J Virol, 88, 14126-37.
WINTER, K., VON KIETZELL, K., HEILBRONN, R., POZZUTO, T., FECHNER, H. & WEGER, S. 2012. Roles of E4orf6 and VA I RNA in adenovirus-mediated stimulation of human parvovirus B19 DNA replication and structural gene expression. J Virol, 86, 5099-109.
Wu Z, Asokan A, Grieger JC, Govindasamy L, Agbandje-McKenna M, Samulski RJ. Single amino acid changes can influence titer, heparin binding, and tissue tropism in different adeno-associated virus serotypes. J Virol. 2006;80(22):11393-11397. doi:10.1128/JVI.01288-06.
Xie, Q., W. Bu, S. Bhatia, J. Hare, T. Somasundaram, A. Azzi, and M. S. Chapman. 2002. The atomic structure of adeno-associated virus (AAV-2), a vector for human gene therapy. Proc Natl Acad Sci U S A 99:10405-10.
ZHANG, H., XIE, J., XIE, Q., WILSON, J. M. & GAO, G. 2009. Adenovirus-adeno-associated virus hybrid for large-scale recombinant adeno-associated virus production. Hum Gene Ther, 20, 922-9.
ZHANG, H. G., WANG, Y. M., XIE, J. F., LIANG, X., HSU, H. C., ZHANG, X., DOUGLAS, J., CURIEL, D. T. & MOUNTZ, J. D. 2001. Recombinant adenovirus expressing adeno-associated virus cap and rep proteins supports production of high-titer recombinant adeno-associated virus. Gene Ther, 8, 704-12.
Li Zhong, Baozheng Li, Cathryn S. Mah, Lakshmanan Govindasamy, Mavis Agbandje-McKenna, Mario Cooper, Roland W. Herzog, Irene Zolotukhin, Kenneth H. Warrington, Kirsten A. Weigel-Van Aken, Jacqueline A. Hobbs, Sergei Zolotukhin, Nicholas Muzyczka, Arun Srivastava. Next generation of adeno-associated virus 2 vectors: Point mutations in tyrosines lead to high-efficiency transduction at lower doses. Proceedings of the National Academy of Sciences Jun 2008, 105 (22) 7827-7832; DOI: 10.1073/pnas.0802866105
ZOLOTUKHIN, S., POTTER, M., HAUSWIRTH, W. W., GUY, J. & MUZYCZKA, N. 1996. A "humanized" green fluorescent protein cDNA adapted for high-level expression in mammalian cells. J Virol, 70, 4646-54.

## Claims

1. A method for producing recombinant adeno-associated virus (rAAV) vectors or AAV virus-like particles, wherein the method comprises infecting a cell with
a) a recombinant adenoviral *rep*-vector (*rep*-rAdV) comprising an expression cassette comprising the AAV *rep* gene operably linked to a promoter, wherein the *rep* gene does not comprise a RIS-Ad and wherein the rep-vector does not encode the AAV Cap proteins, and
b) a recombinant adenoviral *cap*-vector (*cap*-AdV) comprising an expression cassette comprising the AAV *cap* gene operably linked to a promoter that is not the wild-type p40 promoter, wherein said cap-vector does not encode the AAV Rep proteins, and providing
c) a transgene-vector comprising an expression cassette comprising a transgene operably linked to a promoter, wherein the expression cassette is flanked by AAV inverted terminal repeats (ITR),
wherein a) and c) are optional for production of AAV virus-like particles.

2. The method of claim 1, wherein the method is for producing rAAV vectors, wherein the method comprises infecting a cell with
a) a recombinant adenoviral *rep*-vector (*rep*-rAdV) comprising an expression cassette comprising the AAV *rep* gene operably linked to a promoter, wherein the *rep* gene does not comprise a RIS-Ad and wherein the *rep*-vector does not encode the AAV Cap proteins, and
b) a recombinant adenoviral *cap*-vector (*cap*-AdV) comprising an expression cassette comprising the AAV *cap* gene operably linked to a promoter that is not the wild-type p40 promoter, wherein said *cap*-vector does not encode the AAV Rep proteins, and further providing
c) a transgene-vector comprising an expression cassette comprising a transgene operably linked to a promoter, wherein the expression cassette is flanked by AAV ITR.

3. The method of any of claims 1 or 2, wherein the transgene vector is a starter rAAV,
wherein, preferably, the starter rAAV is administered to the cell at a MOI of 10-1500 genomic particles (GP)/cell, preferably at a MOI of 20-50 GP/cell.

4. The method of any of claims 1 or 2, wherein the transgene vector is a starter transfection plasmid.

5. The method of any of claims 1 or 2, wherein the transgene vector is a starter recombinant adenoviral vector.

6. The method of any of the preceding claims, wherein the promoter of the expression cassette of the *rep*-AdV is a heterologous promoter selected from the group comprising a weak promoter, an inducible promoter and a constitutive promoter, wherein preferably the heterologous promoter comprises a TATA-Box, at least one SP1-binding site, and a CCAAT-Box comprising a CTF-binding site,
wherein, optionally, the heterologous promotor is a HSV-TK promotor.

7. The method of any of the preceding claims, wherein the promoter in the expression cassette of the *cap*-AdV is a promoter comprising a TATA-Box, at least one SP1 binding site, a CCAAT-Box and at least one CTF binding site, wherein the promoter preferably is an HSV-TK promoter.

8. The method of any of the preceding claims, wherein the Rep open reading frame is terminated at amino acid 531 by introducing a stop codon at nucleotide position 1911 of SEQ ID NO: 1.

9. The method of any of the preceding claims, wherein the *cap* gene on the second recombinant adenoviral vector encodes the structural proteins VP1, VP2 and VP3 of an AAV serotype selected from the group comprising AAV serotype 1, 2, 3, 4, 5, 6, 7, 8, and 9, wherein, preferably, the structural proteins are from AAV serotype 1, 2, 5, 6, 8 or 9.

10. The method of any of the preceding claims, wherein nucleotides 1701 to 2186 of SEQ ID NO: 1 in the 3' region of the AAV *rep* gene have been recoded to reduce the transcriptional activity of the p40 promoter within the C-terminal part of the Rep open reading frame to at most 30% of that of the wild type p40 promoter,
wherein, preferably, nucleotides 1782 to 1916 of SEQ ID NO: 1 in the 3' region of the AAV *rep* gene have been recoded, optionally, to obtain the sRep sequence of SEQ ID NO: 4.

11. The method of any of the preceding claims, wherein nucleotides 1781-1797 of SEQ ID NO: 1 in the 3' region of the AAV *rep* gene encompassing a GGT-binding site and nucleotides 1851-1856 of SEQ ID NO: 1 in the 3' region of the AAV *rep* gene harboring the p40 transcription start site (TSS) at nucleotide 1853 have been recoded to reduce the transcriptional activity of the p40 promoter in the C-terminal part of the Rep open reading frame to at most 30% of that of the wild type p40 promoter

12. The method of any of the preceding claims, wherein both the *rep*-rAdV and the *cap-*rAdV are administered to the cell at a MOI of 100-2000 GP/cell, preferably at a MOI of 100-500 GP/cell.

13. The method of any of the preceding claims, wherein the *rep*-rAdV and the *cap*-rAdV are first generation recombinant adenoviral vectors.

14. The method of any of the preceding claims, wherein the cell is a human cell, preferably a HEK293 cell.

15. The method of any of the preceding claims, wherein the method is for producing recombinant AAV virus-like particles, wherein the method comprises infecting a cell with a recombinant adenoviral *cap*-vector comprising an expression cassette comprising the AAV *cap* gene operably linked to a promoter that is not the wild-type p40 promoter, wherein said cap-vector does not encode the AAV Rep proteins.

16. A kit or a composition for producing a recombinant adeno-associated virus (rAAV) vector according to any of claims 1-15, comprising
a) a transgene-vector comprising an expression cassette comprising a transgene operably linked to a promoter, wherein the expression cassette is flanked by AAV inverted terminal repeats (ITR), preferably, a starter rAAV vector,
b) a recombinant adenoviral *rep*-vector (*rep*-rAdV) comprising an expression cassette comprising the AAV *rep* gene operably linked to a promoter, wherein the *rep* gene does not comprise a RIS-Ad and wherein the rep-vector does not encode the AAV Cap proteins and
c) a recombinant adenoviral *cap*-vector (*cap*-AdV) comprising an expression cassette comprising the AAV *cap* gene operably linked to a promoter that is not the wild-type p40 promoter, wherein said *cap*-vector does not encode the AAV Rep proteins.
